(19) Europäisches Patentamt European Patent Office Office européen des brevets

(11) **EP 3 878 373 B1**

(12) **EUROPEAN PATENT SPECIFICATION**

(45) Date of publication and mention of the grant of the patent:
**20.09.2023 Bulletin 2023/38**

(21) Application number: **19894584.2**

(22) Date of filing: **06.12.2019**

(51) International Patent Classification (IPC):
**A61B 3/00** *(2006.01)*  **A61B 5/16** *(2006.01)*
**A61B 5/00** *(2006.01)*  **A61B 3/113** *(2006.01)*
**A61B 3/107** *(2006.01)*

(52) Cooperative Patent Classification (CPC):
**A61B 3/107; A61B 3/0058; A61B 3/113;
A61B 5/163; A61B 5/168; A61B 5/742**

(86) International application number:
**PCT/JP2019/047899**

(87) International publication number:
**WO 2020/121975 (18.06.2020 Gazette 2020/25)**

(54) **EVALUATION DEVICE, EVALUATION METHOD, AND EVALUATION PROGRAM**

BEURTEILUNGSVORRICHTUNG, BEURTEILUNGSVERFAHREN UND BEURTEILUNGSPROGRAMM

DISPOSITIF D'ÉVALUATION, PROCÉDÉ D'ÉVALUATION ET PROGRAMME D'ÉVALUATION

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **14.12.2018 JP 2018233988**

(43) Date of publication of application:
**15.09.2021 Bulletin 2021/37**

(73) Proprietors:
• **JVCKENWOOD CORPORATION**
  **Yokohama-shi, Kanagawa 2210022 (JP)**
• **National University Corporation Hamamatsu University School of Medicine Hamamatsu-shi, Shizuoka 431-3192 (JP)**

(72) Inventors:
• **HAKOSHIMA, Shuji**
  **Yokohama-shi, Kanagawa 221-0022 (JP)**
• **TSUCHIYA, Kenji**
  **Hamamatsu-shi, Shizuoka 431-3192 (JP)**

(74) Representative: **Wimmer, Hubert Wagner & Geyer Partnerschaft mbB Patent- und Rechtsanwälte Gewürzmühlstrasse 5 80538 München (DE)**

(56) References cited:
EP-A1- 2 829 221        EP-A1- 2 965 689
WO-A1-2016/052646       WO-A1-2018/216347
JP-A- 2017 099 513      JP-A- 2018 140 007
JP-B2- 5 949 404        JP-B2- 5 971 066
US-A1- 2016 106 354

**Description**

**Field**

The present invention relates to an evaluation device, an evaluation method, and an evaluation program.

**Background**

In recent years, the number of persons with a developmental disability tends to increase. It has already been found that the symptoms can be diminished by detecting the developmental disability early and starting rehabilitation, and thus an effect of adapting to society is high. There is a known technology related to a device used for diagnosis support for determination whether a subject has a characteristic of autism based on a difference of a ratio of a period of time in which the subject is looking at a person facing each other to a period of time in which the subject is looking at other positions (for example, see JP 2011-206542A).

WO 2018 216347 A discloses an evaluating device is provided with: an image data acquiring unit which acquires image data relating to an eyeball of a subject; a point of gaze detecting unit which detects position data relating to a point of gaze of the subject; a display control unit which performs a display operation causing a plurality of objects to be displayed on a display screen, and a hide operation causing the objects to be hidden with a prescribed timing after the display operation; a region setting unit which sets a plurality of corresponding regions of the display screen, corresponding to each object; a determining unit which, on the basis of the point of gaze position data, determines whether or not the point of gaze is present in each corresponding region during a hide period in which the hide operation is being performed; a calculating unit which, on the basis of determination data, obtains the region data indicating each corresponding region, from among the corresponding regions, in which the point of gaze has been detected during the hide period; and an evaluating unit which obtains evaluation data of the subject on the basis of the region data.

JP 2018 140007 A discloses an evaluation device which includes: an image data acquisition part 206 for acquiring image data on a subject's eyeball; a fixation point detection part 214 for detecting position data on the subject's fixation point based on the image data; a display control part 202 for causing a plurality of target videos to be displayed in a display screen; a region setting part 216 for setting a specific region corresponding to each of the plurality of target videos in the display screen; a determination part 218 for determining whether or not the fixation point is present in each specific region based on the position data on the fixation point, and outputting determination data; an arithmetic part 220 for obtaining region data indicating the specific region where the fixation point is first detected of the plurality of specific regions based on the determination data; an evaluation part 224 for obtaining evaluation data on the subject based on the region data; and an output control part 226 for outputting the evaluation data.

JP 2014 068932 A discloses a diagnosis support apparatus which includes a display control unit, a view point detection unit, and a determination unit. The display control unit displays an image on a display unit. The view point detection unit detects a view point of a subject in the image. When it is determined that a degree of concentration is high, the degree of concentration representing a degree where view points are focused on any point on the image, the determination unit determines that the subjects has highly possibly the developmental disability.

US 2016 106354 A discloses a diagnosis supporting device which includes a display, an imaging unit configured to capture a subject, an eye gaze detector configured to detect an eye gaze direction of the subject from a captured image captured by the imaging unit, a gaze point detector configured to detect a gaze point of the subject in a display region of the display, based on the eye gaze direction, an output controller configured to display, on the display, a diagnosis image that includes a person image and a geometric pattern image, and in which at least one of contrast, color density, and a degree of transmittance of the person image or the geometric pattern image is changed over time, and an evaluator configured to calculate an evaluation value of the subject, based on the gaze point detected by the gaze point detector when the diagnosis image is displayed.

**Summary**

A subject diagnosed with autism spectrum disorder (ASD) has general tendencies indicating indifference to existence of others, a strong interest in objects rather than people, an interest in a specific object (obsession). There have been demands for evaluating whether a possibility of the developmental disability is high or low with high accuracy by appropriately detecting these tendencies.

Accordingly, the present disclosure has been conceived in light of the circumstances described above and an object thereof is to provide an evaluation device, an evaluation method, and an evaluation program capable of evaluating whether a possibility of the developmental disability is high or low with high accuracy.

In accordance with the present invention, an An evaluation device, an evaluation method, and an An evaluation program as set forth in the appended claims is provided. In particular, according to one aspect, there is provided an evaluation device comprising: a display configured to display at least one evaluation purpose image; a gaze point detecting unit configured to detect positional data of a gaze point of a subject who observes the display; an area setting unit configured to set at least one determination area in a position corresponding to the at least one evaluation purpose image on the display; a determination unit configured to determine, based on the positional data of the gaze point, whether or not the gaze point is present inside the at least one determination area to define at least one determination value for the at least one determination area; an arithmetic unit configured to calculate, based on a the at least one determination value defined by the determination unit, a number of the at least one determination area in which the gaze point is present for a predetermined period; and an evaluation unit configured to obtain evaluation data of for evaluating a possibility that the subject is a person with a developmental disability based on the number, calculated by the arithmetic unit, of the at least one determination area in which the gaze point is present.

According to one aspect, there is provided an evaluation method comprising: a displaying step of displaying at least one image evaluation purpose on a display; a gaze point detecting step of detecting positional data of a gaze point of a subject who observes the display; an area setting step of setting at least one determination area in a position corresponding to the at least one evaluation purpose image on the display; a determination step of determining, based on the positional data of the gaze point, whether or not the gaze point is present inside the at least one determination area to define at least one determination value for the at least one determination area; an arithmetic step of calculating, based on the at least one determination value defined at the determination step, a number of the at least one determination area in which the gaze point is present for a predetermined period; and an evaluation step of obtaining evaluation data for evaluating a possibility that the subject is a person with a developmental disability based on the number, calculated at the arithmetic step, of the at least one determination area in which the gaze point is present.

According to one aspect, there is provided an evaluation program that causes a computer to execute: a displaying step of displaying at least one evaluation purpose image on a display; a gaze point detecting step of detecting positional data of a gaze point of a subject who observes the display; an area setting step of setting at least one determination area in a position corresponding to the at least one evaluation purpose image on the display; a determination step of determining, based on the positional data of the gaze point, whether or not the gaze point is present inside the at least one determination area to define at least one determination value for the at least one determination area; an arithmetic step of calculating, based on the at least one determination value defined at the determination step, a number of the at least one determination area in which the gaze point is present for a predetermined period; and an evaluation step of obtaining evaluation data for evaluating a possibility that the subject is a person with a developmental disability based on the number, calculated at the arithmetic step, of the at least one determination area in which the gaze point is present.

According to the present disclosure, it is possible to provide an evaluation device, an evaluation method, and an evaluation program capable of evaluating whether a possibility of the developmental disability is high or low with high accuracy.

## Brief Description of Drawings

FIG. 1 is a perspective view schematically illustrating an example of a line-of-sight detecting device according to one embodiments;

FIG. 2 is a diagram illustrating an example of a hardware configuration of the line-of-sight detecting device according to the embodiment;

FIG. 3 is a functional block diagram illustrating an example of the line-of-sight detecting device according to the embodiment;

FIG. 4 is a schematic diagram illustrating a method for calculating positional data of a corneal curvature center according to the embodiment;

FIG. 5 is a schematic diagram illustrating a method for calculating positional data of a corneal curvature center according to the embodiment;

FIG. 6 is a schematic diagram illustrating an example of a calibration process according to the embodiment;

FIG. 7 is a schematic diagram illustrating an example of a gaze point detecting process according to the embodiment;

FIG. 8 is a diagram illustrating an example of an evaluation purpose image;

FIG. 9 is a diagram illustrating an example of determination areas that are set in the evaluation purpose image illustrated in FIG. 8;

FIG. 10 is a diagram illustrating another example of the evaluation purpose image;

FIG. 11 is a diagram illustrating an example of determination areas that are set in the evaluation purpose image illustrated in FIG. 10;

FIG. 12 is a diagram illustrating an example of gaze points for the evaluation purpose image illustrated in FIG. 8;

FIG. 13 is a diagram illustrating another example of gaze points for the evaluation purpose image illustrated in FIG. 8;

FIG. 14 is a diagram illustrating an example of gaze points for the evaluation purpose image illustrated in FIG. 10;
FIG. 15 is a diagram illustrating another example of gaze points for the evaluation purpose image illustrated in FIG. 10;
FIG. 16 is a diagram illustrating another example of an evaluation purpose image;
FIG. 17 is a diagram illustrating an example of determination areas that are set in the evaluation purpose image illustrated in FIG. 16; and
FIG. 18 is a flowchart illustrating an example of an evaluation method according to the embodiment.

**Description of Embodiments**

Preferred embodiments of an evaluation device, an evaluation method, and an evaluation program according to the present disclosure will be described based on the drawings. Furthermore, the present invention is not limited to the embodiments. Furthermore, the components described in the embodiments include one that can easily be replaced by those skilled in the art or one that is substantially identical.

In a description below, the positional relationships among components will be described by setting a three-dimensional global coordinate system. It is assumed that a direction parallel to a first axis of a predetermined plane is defined as an X-axis direction, a direction parallel to a second axis of the predetermined plane orthogonal to the first axis is defined as a Y-axis direction, and a direction parallel to a third axis that is orthogonal to each of the first axis and the second axis is defined as a Z-axis direction. The predetermined plane includes an XY plane.

Line-of-Sight Detecting Unit

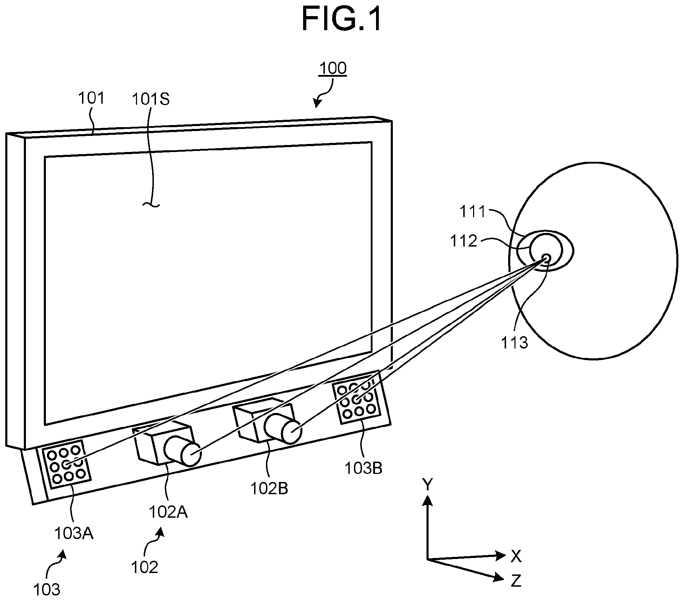

FIG. 1 is a perspective view schematically illustrating an example of a line-of-sight detecting device 100 according to a first embodiment. The line-of-sight detecting device 100 is used as an evaluation device that evaluates whether a possibility of a developmental disability is high or low. As illustrated in FIG. 1, the line-of-sight detecting device 100 includes a display device 101, a stereo camera device 102, and an illuminating device 103.

The display device 101 includes a flat panel display, such as a liquid crystal display (LCD) or an organic electro-luminescence (EL) display (OLED). In the embodiment, the display device 101 includes a display 101S. The display 101S displays an image. In the embodiment, the display 101S displays an index for evaluating, for example, a visual performance of a subject. The display 101S is substantially parallel to the XY plane. The X-axis direction corresponds to a horizontal direction of the display 101S, the Y-axis direction corresponds to a vertical direction of the display 101S, and the Z-axis direction corresponds to a depth direction orthogonal to the display 101S.

The stereo camera device 102 includes a first camera 102A and a second camera 102B. The stereo camera device 102 is arranged below the display 101S of the display device 101. The first camera 102A and the second camera 102B are arranged in the X-axis direction. The first camera 102A is arranged in a negative X direction relative to the second camera 102B. Each of the first camera 102A and the second camera 102B includes an infrared camera and includes an optical system capable of transmitting near-infrared light with a wavelength of, for example, 850 (nm) and an image sensor capable of receiving the near-infrared light.

The illuminating device 103 includes a first light source 103A and a second light source 103B. The illuminating device 103 is arranged below the display 101S of the display device 101. The first light source 103A and the second light source 103B are arranged in the X-axis direction. The first light source 103A is arrange in a negative direction relative to the first camera 102A. The second light source 103B is arranged in a positive direction relative to the second camera 102B. Each of the first light source 103A and the second light source 103B includes a light emitting diode (LED) light source and is able to emit near-infrared light with a wavelength of, for example, 850 (nm). Furthermore, the first light source 103A and the second light source 103B may also be arranged between the first camera 102A and the second camera 102B.

The illuminating device 103 emits near-infrared light that is detection light and illuminates an eyeball 111 of a subject. The stereo camera device 102 captures an image of a part of the eyeball 111 (hereinafter, referred to as an "eyeball" including the part of the eyeball) by the second camera 102B when the eyeball 111 is irradiated with the detection light emitted from the first light source 103A and captures an image of the eyeball 111 by the first camera 102A when the eyeball 111 is irradiated with the detection light emitted from the second light source 103B.

A frame synchronization signal is output from at least one of the first camera 102A and the second camera 102B. The first light source 103A and the second light source 103B output detection light based on the frame synchronization signal. The first camera 102A captures image data of the eyeball 111 when the eyeball 111 is irradiated with the detection light emitted from the second light source 103B. The second camera 102B captures image data of the eyeball 111 when the eyeball 111 is irradiated with the detection light emitted from the first light source 103A.

When the eyeball 111 is irradiated with the detection light, a part of the detection light is reflected at a pupil 112 and the light from the pupil 112 is incident into the stereo camera device 102. Furthermore, when the eyeball 111 is irradiated with the detection light, a corneal reflection image 113 that is a virtual image of a cornea is formed on the eyeball 111 and the light from the corneal reflection image 113 is incident into the stereo camera device 102.

By appropriately setting a relative position between a set of the first camera 102A and the second camera 102B and a set of the first light source 103A and the second light source 103B, an intensity of the light incident from the pupil 112 to the stereo camera device 102 is reduced and an intensity of the light incident from the corneal reflection image 113 to the stereo camera device 102 is increased. That is, the image of the pupil 112 captured by the stereo camera device 102 has a low luminance and the image of the corneal reflection image 113 has a high luminance. The stereo camera device 102 can detect a position of the pupil 112 and a position of the corneal reflection image 113 based on the luminance of the image captured.

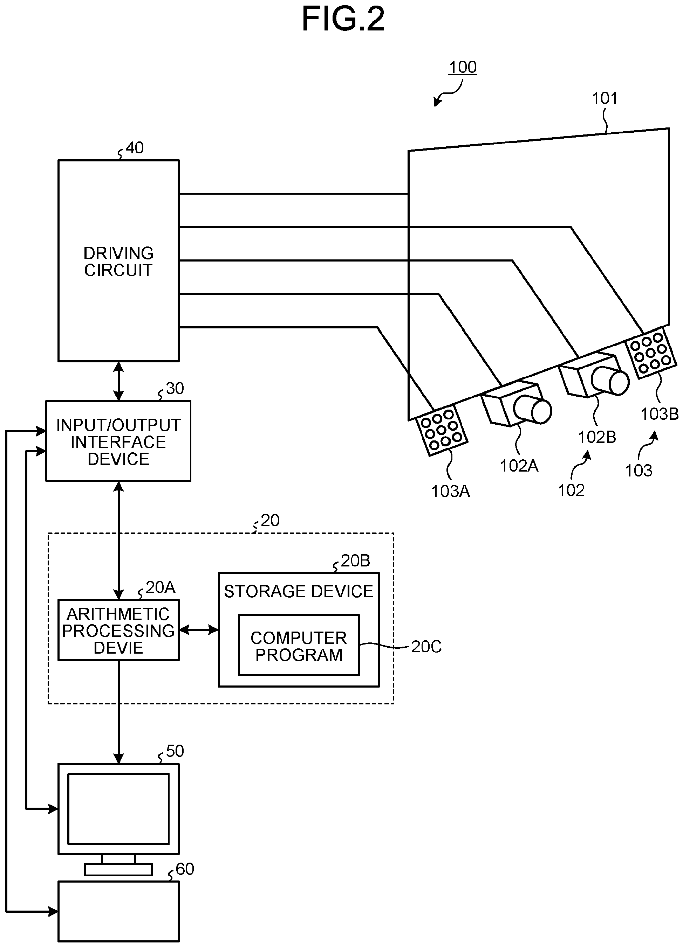

FIG. 2 is a diagram illustrating an example of a hardware configuration of the line-of-sight detecting device 100 according to the embodiment. As illustrated in FIG. 2, the line-of-sight detecting device 100 includes the display device 101, the stereo camera device 102, the illuminating device 103, a computer system 20, an input/output interface device 30, a driving circuit 40, an output device 50, and an input device 60.

The computer system 20, the driving circuit 40, the output device 50, and the input device 60 perform data communication via the input/output interface device 30. The computer system 20 includes an arithmetic processing device 20A and a storage device 20B. The arithmetic processing device 20A includes a microprocessor, such as a central processing unit (CPU). The storage device 20B includes a memory, such as a read only memory (ROM) and a random access memory (RAM), or storage. The arithmetic processing device 20A performs an arithmetic processing in accordance with a computer program 20C that is stored in the storage device 20B.

The driving circuit 40 generates a driving signal and outputs the driving signal to the display device 101, the stereo camera device 102, and the illuminating device 103. Furthermore, the driving circuit 40 supplies the image data of the eyeball 111 captured by the stereo camera device 102 to the computer system 20 via the input/output interface device 30.

The output device 50 includes a display, such as a flat panel display. Furthermore, the output device 50 may also include a printer. The input device 60 generates input data by being operated. The input device 60 includes a keyboard or a mouse for a computer system. Furthermore, the input device 60 may also include a touch sensor arranged on the display of the output device 50 as a display.

In the embodiment, the display device 101 and the computer system 20 are separated devices. Furthermore, the display device 101 and the computer system 20 may also be integrated. For example, if the line-of-sight detecting device 100 includes a tablet type personal computer, the computer system 20, the input/output interface device 30, the driving circuit 40, and the display device 101 may also be mounted on the tablet type personal computer.

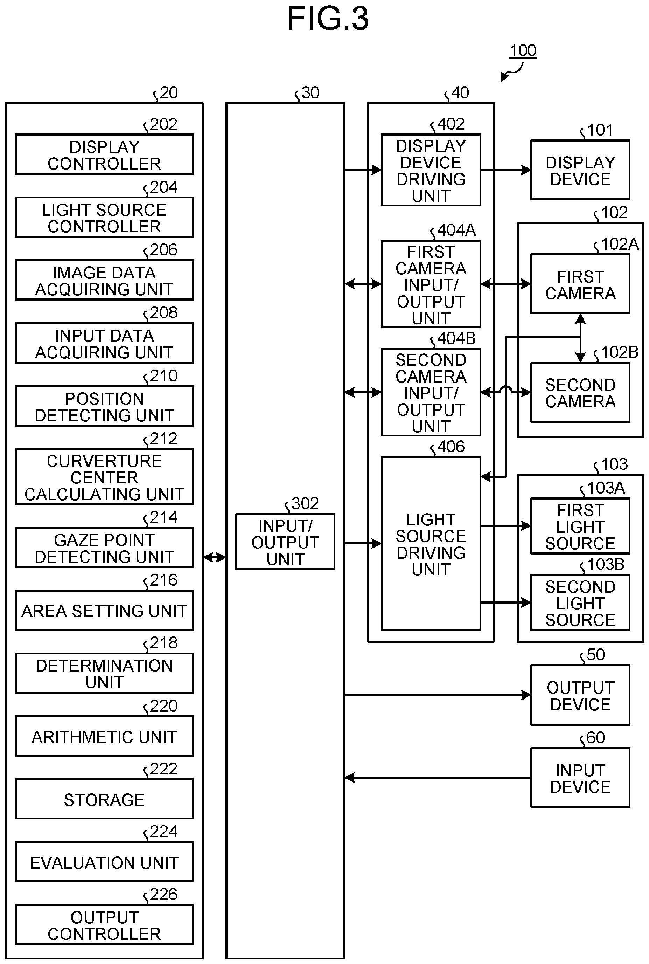

FIG. 3 is a functional block diagram illustrating an example of the line-of-sight detecting device 100 according to the embodiment. As illustrated in FIG. 3, the input/output interface device 30 includes an input/output unit 302.

The driving circuit 40 includes a display driving unit 402 that generates a driving signal for driving the display device 101 and outputs the driving signal to the display device 101, a first camera input/output unit 404A that generates a driving signal for driving the first camera 102A and outputs the driving signal to the first camera 102A, a second camera input/output unit 404B that generates a driving signal for driving the second camera 102B and outputs the driving signal to the second camera 102B, and a light source driving unit 406 that generates a driving signal for driving the first light source 103A and the second light source 103B and outputs the driving signal to the first light source 103A and the second light source 103B. Furthermore, the first camera input/output unit 404A supplies the image data of the eyeball 111 captured by the first camera 102A to the computer system 20 via the input/output unit 302. The second camera input/output unit 404B supplies the image data of the eyeball 111 captured by the second camera 102B to the computer system 20 via the input/output unit 302.

The computer system 20 controls the line-of-sight detecting device 100. The computer system 20 includes a display controller 202, a light source controller 204, an image data acquiring unit 206, an input data acquiring unit 208, a position detecting unit 210, a curvature center calculating unit 212, a gaze point detecting unit 214, an area setting unit 216, a determination unit 218, an arithmetic unit 220, a storage 222, an evaluation unit 224, and an output controller 226. The function of the computer system 20 is performed by the arithmetic processing device 20A and the storage device 20B.

The display controller 202 controls the display driving unit 402 such that an evaluation purpose image to be visually recognized by the subject is displayed on the display 101S of the display device 101. The evaluation purpose image includes a still image and a moving image. For example, multiple evaluation purpose images are prepared. The display controller 202 sequentially displays the multiple evaluation purpose images on the display device 101. Furthermore, the display controller 202 may also allow the display device 101 to display an eye-catching video that is used to position a gaze point P at a desired position on the display 101S.

The light source controller 204 controls the light source driving unit 406 and controls an operation state of the first light source 103A and the second light source 103B. The light source controller 204 controls the first light source 103A and the second light source 103B such that the first light source 103A and the second light source 103B emit the detection light at different timings.

The image data acquiring unit 206 acquires, from the stereo camera device 102 via the input/output unit 302, the image data of the eyeball 111 of the subject captured by the stereo camera device 102 that includes the first camera 102A and the second camera 102B.

The input data acquiring unit 208 acquires, from the input device 60 via the input/output unit 302, the input data generated by an operation of the input device 60.

The position detecting unit 210 detects positional data of the pupil center based on the image data of the eyeball 111 acquired by the image data acquiring unit 206. Furthermore, the position detecting unit 210 detects positional data of the corneal reflection center based on the image data of the eyeball 111 acquired by the image data acquiring unit 206. The pupil center is a center of the pupil 112. The corneal reflection center is a center of the corneal reflection image 113. The position detecting unit 210 detects, for each of the left and right eyeballs 111 of the subject, the positional data of the pupil center and the positional data of the corneal reflection center.

The curvature center calculating unit 212 calculates positional data of a corneal curvature center of the eyeball 111 based on the image data of the eyeball 111 acquired by the image data acquiring unit 206.

The gaze point detecting unit 214 detects positional data of the gaze point P of the subject based on the image data of the eyeball 111 acquired by the image data acquiring unit 206. In the present embodiment, the positional data of the gaze point P indicates the positional data of an intersection point between a line-of-sight vector of the subject that is defined by the three-dimensional global coordinate system and the display 101S of the display device 101. The gaze point detecting unit 214 detects a line-of-sight vector of each of the right and left eyeballs 111 of the subject based on the positional data of the pupil center and the positional data of the corneal curvature center that are acquired from the image data of the eyeball 111. After the line-of-sight vector has been detected, the gaze point detecting unit 214 detects the positional data of the gaze point P indicating the intersection point between the line-of-sight vector and the display 101S.

The area setting unit 216 sets a determination area for the evaluation purpose image displayed on the display 101S of the display device 101. The area setting unit 216 sets the determination area to a person, an object, a pattern, and the like included in the evaluation purpose image. In the embodiment, the area setting unit 216 sets the determination area to at least a part of an evaluation purpose image on the display 101S. It is preferable that the number of determination areas to be set be plural. If a natural picture and a geometric image are displayed on the display 101S, the area setting unit 216 may also set the determination area to, for example, at least a part of the area in which the natural picture is displayed and at least a part of the area in which the geometric image is displayed. The determination area is preferably set with respect to an object included in an evaluation purpose video. When the determination area is set with respect to a natural picture, for example, the determination area is preferably set to each of the objects, such as a person or a face of a person. The determination area is not displayed on the display 101S. The shape of the determination area is not limited and, for example, a rectangular shape, a circular shape, an elliptical shape, a polygonal shape, and the like may also be used. Each size of the multiple determination areas may also be different. The multiple determination areas may also be set in an overlapping manner. The determination area is preferably set in an area of the evaluation purpose image in which a tendency to be gazed varies between the subject who is highly likely to be ASD and the subject who is less likely to be ASD.

The determination unit 218 determines whether the gaze point P is present inside the determination area based on the positional data of the gaze point P and outputs the determination data. For example, the determination unit 218 outputs determination data in which, when it is determined that the gaze point P is present inside the determination area, a determination value of the determination area is defined as "1", whereas, when it is determined that the gaze point P is not present, the determination value of the determination area is defined as "0". When the determination unit 218 determines that the gaze point P is present inside the determination area, the determination unit 218 defines the determination value as "1" regardless of a gaze period and the number of times the subject gazes the determination area. The determination unit 218 defines the determination value as "1" regardless of whether or not the gaze period is long or short. The determination unit 218 defines the determination value as "1" even when the gaze point P moves between the determination area and the other area and is determined to be present inside the determination area more than once. The determination unit 218 determines whether the gaze point P is present inside the determination area at, for example, every fixed time. For example, a period (for example, every 20 (msec)) of a frame synchronization signal that is output from the first camera 102A and the second camera 102B can be applied to the fixed time.

The arithmetic unit 220 calculates the total number of the determination areas in each of which the gaze point P is present for a predetermined period based on the determination data by the determination unit 218. In other words, the arithmetic unit 220 calculates the number of determination areas included in the evaluation purpose image gazed by the subject for the predetermined period. The arithmetic unit 220 accumulates the determination value of each of the determination areas and calculates the total number of determination areas in each of which the gaze point P is present.

The arithmetic unit 220 includes a management timer that manages a playback time of a video and a detection timer that detects an elapsed time from displaying the video on the display 101S.

The evaluation unit 224 obtains evaluation data of the subject based on the total number of the determination areas in each of which the gaze point P is present calculated by the arithmetic unit 220. The evaluation data is data for evaluating the number of times the subject gazed the determination areas displayed on the display 101S by a display operation. The evaluation unit 224 evaluates the number of gazed determination areas regardless of, for example, the period of time for which the subject has gazed the determination area and the number of times the subject has gazed the determination area. The evaluation unit 224 may also obtain the evaluation data by applying weighting to each of the determination areas in each of which the gaze point P is present. The evaluation unit 224 determines whether the evaluation data is greater than or equal to a predetermined threshold to determine the evaluation data.

The storage 22 stores therein the determination data and the evaluation data described above. Furthermore, the storage 222 stores therein an evaluation program that causes a computer to execute a process of displaying an image, a process of detecting a position of the gaze point P of the subject who observes the display, a process of setting the determination area on the display, a process of outputting the determination data by determining whether the gaze point P is present inside the determination area based on the positional data of the gaze point P, a process of obtaining the evaluation data of the subject based on the determination data, and a process of outputting the evaluation data.

The output controller 226 outputs the data to at least one of the display device 101 and the output device 50.

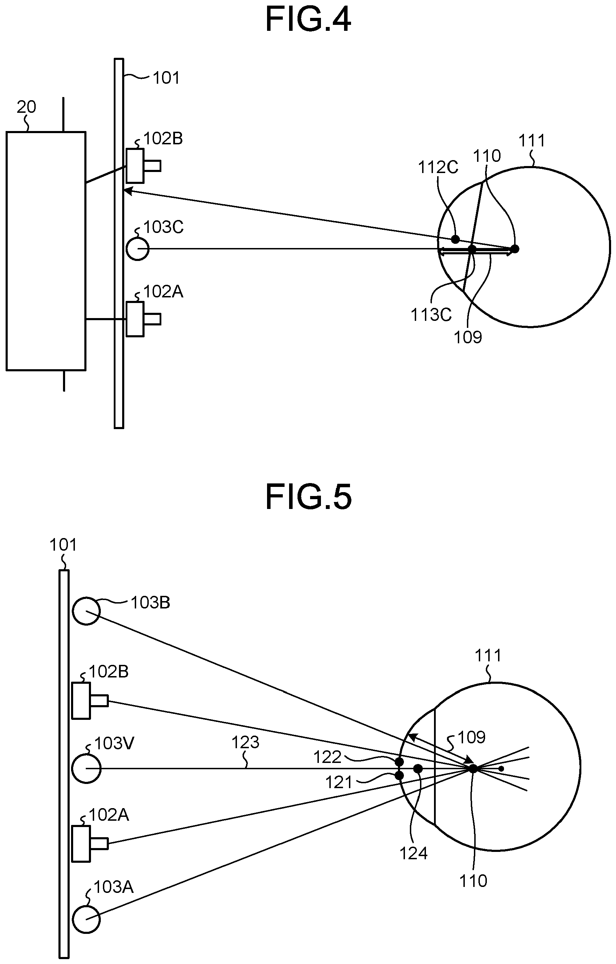

In the following, an outline of processes performed by the curvature center calculating unit 212 according to the embodiment will be described. The curvature center calculating unit 212 calculates the positional data of the corneal curvature center of the eyeball 111 based on the image data of the eyeball 111. Each of FIG. 4 and FIG. 5 is a schematic diagram illustrating a calculation method of positional data of a corneal curvature center 110 according to the embodiment. FIG. 4 illustrates an example in which the eyeball 111 is illuminated by a light source 103C. FIG. 5 illustrates an example in which the eyeball 111 is illuminated by the first light source 103A and the second light source 103B.

First, the example illustrated in FIG. 4 will be described. The light source 103C is arranged between the first camera 102A and the second camera 102B. A pupil center 112C is a center of the pupil 112. A corneal reflection center 113C is a center of the corneal reflection image 113. In FIG. 4, the pupil center 112C indicates a pupil center when the eyeball 111 is illuminated by the single light source 103C. The corneal reflection center 113C indicates a corneal reflection center when the eyeball 111 is illuminated by the single light source 103C. The corneal reflection center 113C is present on a straight line connecting the light source 103C and a corneal curvature center 110. The corneal reflection center 113C is positioned at a middle point between a cornea surface and the corneal curvature center 110. A corneal curvature radius 109 is a distance between the cornea surface and the corneal curvature center 110. Positional data of the corneal reflection center 113C is detected by the stereo camera device 102. The corneal curvature center 110 is present on a straight line connecting the light source 103C and the corneal reflection center 113C. The curvature center calculating unit 212 calculates, as the positional data of the corneal curvature center 110, positional data of a position which is located at a predetermined distance from the corneal reflection center 113C on the straight line. The predetermined value is a value that is determined in advance from a curvature radius value of a general cornea or the like and is stored in the storage 222.

In the following, the example illustrated in FIG. 5 will be described. In the embodiment, a set of the first camera 102A and the second light source 103B and a set of the second camera 102B and the first light source 103A are arranged at bilaterally symmetrical positions with respect to a straight line that passes through an intermediate position between the first camera 102A and the second camera 102B. It is assumed that a virtual light source 103V is present at the intermediate position between the first camera 102A and the second camera 102B. A corneal reflection center 121 indicates a corneal reflection center in an image that is obtained by capturing the eyeball 111 by the second camera 102B. A corneal reflection center 122 indicates a corneal reflection center in an image that is obtained by capturing the eyeball 111 by the first camera 102A. A corneal reflection center 124 indicates a corneal reflection center associated with the virtual light source 103V. Positional data of the corneal reflection center 124 is calculated based on positional data of the corneal reflection center 121 and positional data of the corneal reflection center 122 that are captured by the stereo camera device 102. The stereo camera device 102 detects the positional data of the corneal reflection center 121 and the positional data of the corneal reflection center 122 in the three-dimensional local coordinate system that is defined in the stereo camera device 102. A camera calibration using a stereo calibration method is performed in advance on the stereo camera device 102, and a transformation parameter for transforming the three dimensional local coordinate system of the stereo camera device 102 into the three-dimensional global coordinate system is calculated. The transformation parameter is stored in the storage 222. The curvature center calculating unit 212 transforms the positional data of the corneal reflection center 121 and the positional data of the corneal reflection center 122 captured by the stereo camera device 102 into the positional data in the three-dimensional global coordinate system by using the transformation parameter. The curvature center calculating unit 212 calculates the positional data of the corneal reflection center 124 in the three-dimensional global coordinate system based on the positional data of the corneal reflection center 121 and the positional data of the corneal reflection center 122 that are defined in the three-dimensional global coordinate system. The corneal curvature center 110 is present on a straight line 123 connecting the virtual light source 103V and the corneal reflection center 124. The curvature center calculating unit 212 calculates, as the positional data of the corneal curvature center 110, positional data of a position which is located at a predetermined distance from the corneal reflection center 124 on the straight line 123. The predetermined value is a value that is determined in advance from a curvature radius value of a general cornea or the like and is stored in the storage 222.

In this way, even when two light sources are present, the corneal curvature center 110 is calculated by the same method as the method that is used when a single light source is present.

The corneal curvature radius 109 corresponds to a distance between the cornea surface and the corneal curvature center 110. Accordingly, the corneal curvature radius 109 is calculated by calculating the positional data of the cornea surface and the positional data of the corneal curvature center 110.

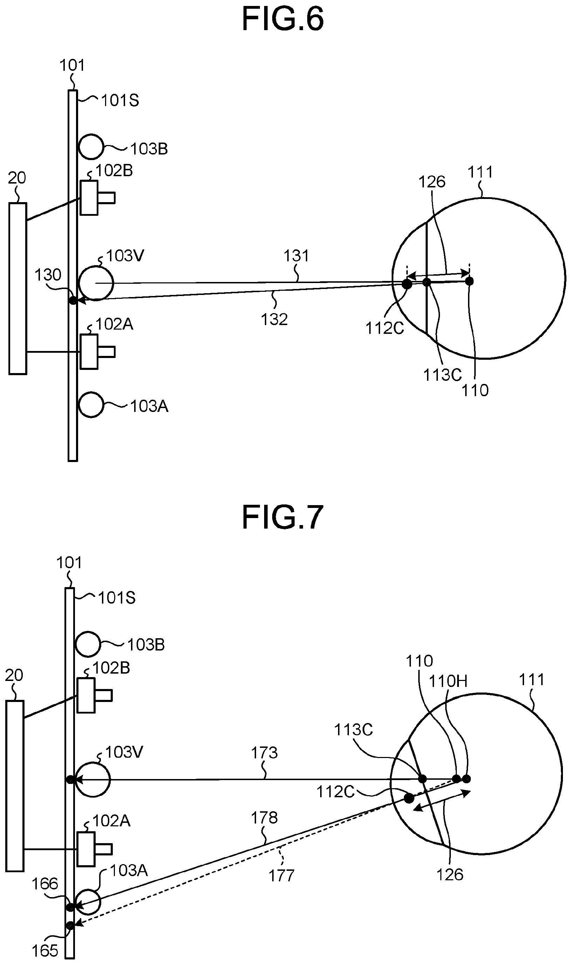

In the following, an example of a line-of-sight detecting method according to the embodiment will be described. FIG. 6 is a schematic diagram illustrating an example of a calibration process according to the embodiment. In the calibration process, a target position 130 is set in order to allow the subject to gaze steadily. The target position 130 is defined in the three-dimensional global coordinate system. In the embodiment, the target position 130 is set at, for example, a middle position of the display 101S of the display device 101. Furthermore, the target position 130 may also be set at an edge position of the display 101S. The output controller 226 displays a target image at the set target position 130. A straight line 131 is a straight line connecting the virtual light source 103V and the corneal reflection center 113C. A straight line 132 is a straight line connecting the target position 130 and the pupil center 112C. The corneal curvature center 110 is an intersection point between the straight line 131 and the straight line 132. The curvature center calculating unit 212 can calculate the positional data of the corneal curvature center 110 based on the positional data of the virtual light source 103V, the positional data of the target position 130, the positional data of the pupil center 112C, and the positional data of the corneal reflection center 113C.

In the following, a gaze point detecting process performed by the gaze point detecting unit 214 will be described. The gaze point detecting process is performed after the calibration process. The gaze point detecting unit 214 calculates a line-of-sight vector of the subject and positional data of the gaze point P based on the image data of the eyeball 111. FIG. 7 is a schematic diagram illustrating an example of the gaze point detecting process according to the embodiment. In FIG. 7, a gaze point 165 indicates a gaze point P that is obtained from the corneal curvature center calculated using a general curvature radius value. A gaze point 166 indicates a gaze point P that is obtained from the corneal curvature center calculated using a distance 126 obtained in the calibration process. The pupil center 112C indicates the pupil center calculated in the calibration process, and the corneal reflection center 113C indicates the corneal reflection center calculated in the calibration process. A straight line 173 is a straight line connecting the virtual light source 103V and the corneal reflection center 113C. The corneal curvature center 110 corresponds to a position of the corneal curvature center that is calculated from a general curvature radius value. The distance 126 is a distance between the pupil center 112C and the corneal curvature center 110 calculated in the calibration process. A corneal curvature center 110H indicates a corrected position of the corneal curvature center that has been corrected by using the distance 126. The corneal curvature center 110H is obtained under a condition that the corneal curvature center 110 is present on the straight line 173 and the distance between the pupil center 112C and the corneal curvature center 110 is the distance 126. Accordingly, a line of sight 177 that is calculated in a case of using the general curvature radius value is corrected to a line of sight 178. Furthermore, the gaze point P on the display 101S of the display device 101 is corrected from the gaze point 165 to the gaze point 166.

Evaluation Method

In the following, the evaluation method according to the embodiment will be described. In the evaluation method according to the embodiment, a developmental disability is evaluated as a visual performance of the subject by using the line-of-sight detecting device 100 described above.

The display controller 202 displays an evaluation purpose image on the display 101S. Before the display controller 202 displays the evaluation purpose image, the display controller 202 may also display a eye-catching video on the display 101S and locate the gaze point P of the subject at a desired position on the display 101S.

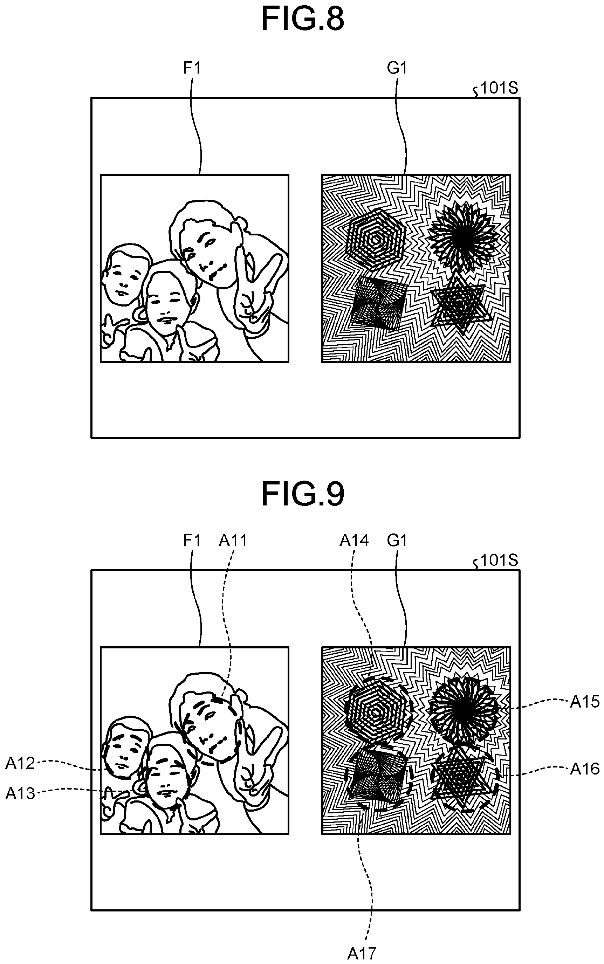

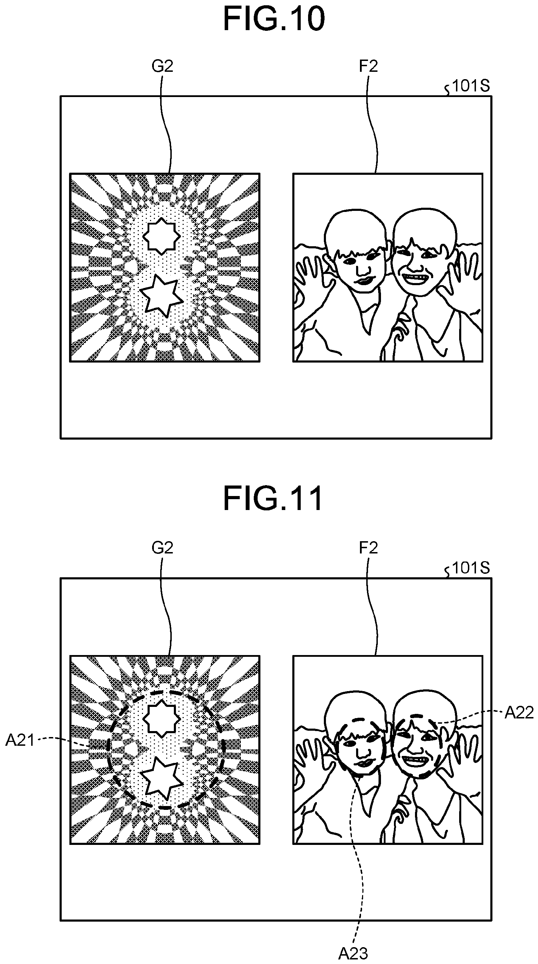

First, examples of the evaluation purpose images will be described by using FIG. 8 to FIG. 11. FIG. 8 is a diagram illustrating an example of an evaluation purpose image. FIG. 9 is a diagram illustrating an example of a determination area that is set in the evaluation purpose image illustrated in FIG. 8. FIG. 10 is a diagram illustrating another example of an evaluation purpose image. FIG. 11 is a diagram illustrating an example of a determination area that is set in the evaluation purpose image illustrated in FIG. 10.

The evaluation purpose images indicated here include natural pictures and geometric images. This is because a person with a developmental disability prefers a geometric image video to a natural picture video. Furthermore, a subject diagnosed with ASD has general tendencies indicating indifference to existence of others, a strong interest in objects rather than people, an interest in a specific object (obsession). The natural picture may also be an image that is other than a geometric image and also that is a natural object or that evokes a natural object. For example, it may also be possible to use, as a natural picture, an image (a still image, a moving image) of a person, an animal, a plant, a natural landscape, or the like captured by a camera. Furthermore, it may also be possible to use, as a natural picture, a character image (a still image, a moving image) obtained by imitating a person, an animal, or the like.

The evaluation purpose image illustrated in FIG. 8 displays a people video as a natural image F1 on a left side and displays a geometric image G1 on a right side. Here, the natural image F1 and the geometric image G1 are designed to have a similar color, brightness, motion, and the like.

Furthermore, as illustrated in FIG. 9, in order to perform evaluation, in the evaluation purpose image, a determination area A11, a determination area A12, and a determination area A13, each having a circular shape, are set in the vicinity of faces of corresponding persons, and a determination area A14, a determination area A15, a determination area A16, and a determination area A17, each having a circular shape, are set in portions of geometric patterns. The determination area A11, the determination area A12, the determination area A13, the determination area A14, the determination area A15, the determination area A16, and the determination area A17 are not displayed on the display 101S. By calculating an evaluation value as evaluation data obtained by accumulating determination values indicating whether these determination areas are gazed at by the subject, an evaluation whether the subject is close to be in a typical development or whether the subject is highly likely or less likely to be a developmental disability is performed.

The evaluation purpose image illustrated in FIG. 10 displays, in comparison to FIG. 8, a natural image F2 on the right side and a geometric image G2 on the left side. As illustrated in FIG. 11, a determination area A22 and a determination area A23 each having a circular shape are set in the vicinity of faces of corresponding persons, and a determination area A21 having a circular shape is set in a portion of a geometric pattern. This is because, when these evaluation purpose images are shown, there may be a case in which the subject begins to look at the images starting from the right side, a case in which the subject looks at the image on the left side for a longer period, or the like due to his/her habit. Therefore, in order to reduce this influence, there is a method (counterbalance) for producing videos that have different right and left arrangements in the same number and showing these videos. However, it is difficult to completely eliminate this influence.

In general, an evaluation is performed by showing multiple evaluation purpose images, such as combination patterns of multiple natural pictures and geometric images, to the subject and comprehensively determining whether a possibility of the developmental disability is high of low. This is performed in order to exclude contingency and reduce an influence of a case in which the subject looks at the images with a different characteristic from the characteristic of the developmental disability when preferred image for the subject are displayed. Thus, for example, the evaluation purpose images are presented to be looked at by the subject in the order from FIG. 8 to FIG. 10.

When the subject is allowed to visually recognize the evaluation purpose images described above, if the subject is a person with the developmental disability, the subject tends to be more interested in a geometric image than a people video. Furthermore, if the subject is a person with the developmental disability, the subject tends to be interested in a specific object. In this case, the gaze point P moves to the geometric image, and the subject tends to more gaze the geometric image than the people image. In contrast, if the subject is not a person with the developmental disability, since there is no tendency of an interest in a specific object, a tendency of a strong interest in a specific image is not observed. In this case, the gaze point P often moves in the images and many areas are tend to be gazed at by the subject.

Accordingly, it is possible to evaluate the subject by performing, for example, a following procedure. In the embodiment, a gaze determination for the determination areas that are set in the evaluation purpose images is performed by showing the evaluation images on the display 101S and, at the same time, measuring the gaze point P of the subject. When the gaze point P of the subject enters the determination area, it is determined that the subject gazed at an corresponding gaze target, and then, the total number of the determination areas gazed at by the subject for the multiple evaluation purpose images is set to an evaluation value. Because an ASD subject tends to have a relatively low evaluation value due to the above described reason, it is possible to perform ASD diagnosis support with high accuracy.

If the gaze point P enters the determination area, the determination unit 218 defines the determination value of the corresponding determination area to "1". The arithmetic unit 220 calculates the total number by accumulating the number of the determination area in which the gaze point P is present.

In the embodiment, the evaluation unit 224 can determine a possibility of ASD by accumulating, for example, the determination values of the determination areas in the multiple images. The evaluation unit 224 can evaluate that the subject does not have a tendency to be interested in a specific object as the evaluation value is greater. Furthermore, in this case, it is possible to evaluate that the subject is less likely to be a person with the developmental disability. The evaluation unit 224 can evaluate that the subject has a tendency to be more interested in the geometric image or has a tendency to be interested in a specific object as the evaluation value is smaller. Furthermore, in this case, it is possible to evaluate that the subject is highly likely to be a person with the developmental disability.

Here, the determination value in each of the determination areas in the evaluation purpose image illustrated in FIG. 8 is denoted by X[determination area]. For example, the determination value in the determination area A11 is denoted by X[A11], and the determination value in the determination area A14 is denoted by X[A14]. Furthermore, the determination value in the determination area in which the gaze point P is not present is denoted by "0", and the determination value of the determination area in which the gaze point P is present is denoted by "1".

In this case, an evaluation value ANS1 with respect to the evaluation purpose image illustrated in FIG. 8 is obtained by accumulating the determination values of the determination areas, and is represented by

$$ANS1=X[A11]+X[A12]+X[A13]+X[A14]+X[A15]+X[A16]+X[A17].$$

An evaluation value ANS2 with respect to the evaluation purpose image illustrated in FIG. 10 is obtained by accumulating the determination values of the determination areas, and is represented by

$$ANS2=X[A21]+X[A22]+X[A23].$$

Based on these, by adding the evaluation value ANS1 for the evaluation purpose image illustrated in FIG. 8 and the evaluation value ANS2 for the evaluation purpose image illustrated in FIG. 10, the evaluation value ANS of the subject is represented by, for example, ANS=ANS1+ANS2.

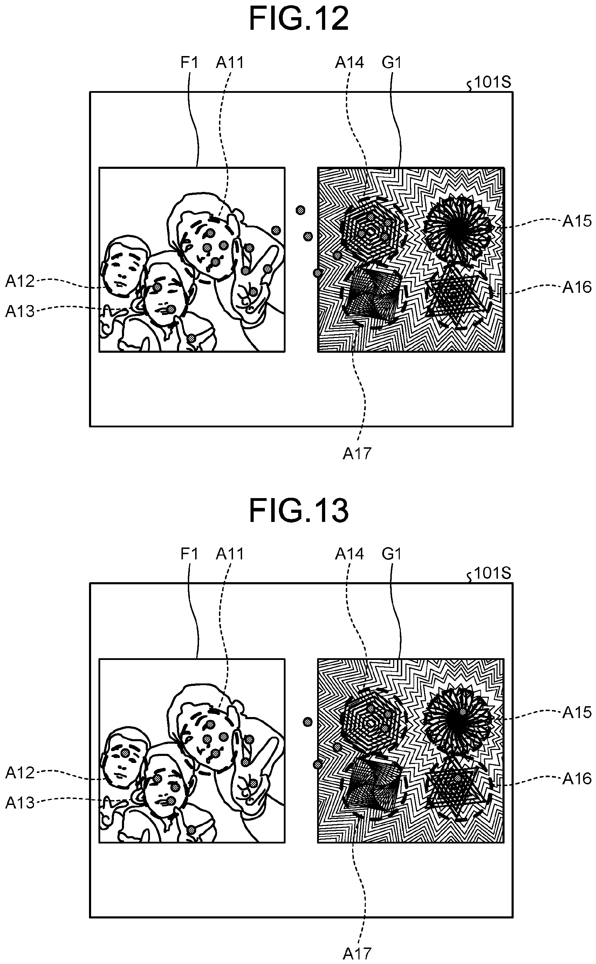

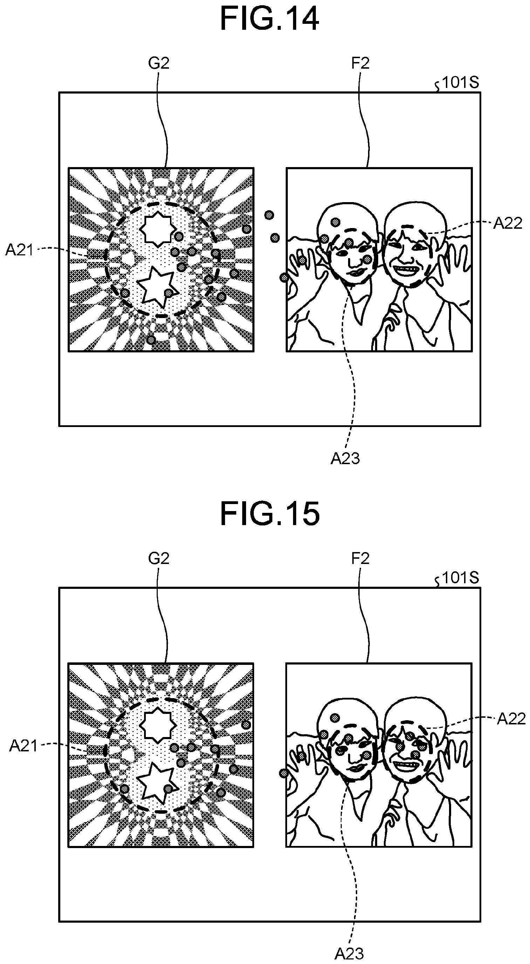

The evaluation value ANS of a subject A will be described by using FIG. 12 and FIG. 14. FIG. 12 is a diagram illustrating an example of the gaze point with respect to the evaluation purpose image illustrated in FIG. 8. FIG. 14 is a diagram illustrating an example of the gaze point with respect to the evaluation purpose image illustrated in FIG. 10.

The evaluation value ANS1 of the subject A with respect to the evaluation purpose image illustrated in FIG. 8 is calculated to be ANS1=1+0+1+1+0+0+0=3.

The evaluation value ANS2 of the subject A with respect to the evaluation purpose image illustrated in FIG. 10 is calculated to be

$$ANS2=1+0+1=2.$$

Based on these, the evaluation value ANS of the subject A is calculated to be ANS=3+2=5.

For example, when a threshold for determining whether or not a possibility of ASD is high is set to be "7", since the evaluation value ANS of the subject A is not greater than or equal to the threshold, it is determined that "a possibility of ASD is high".

The evaluation value ANS of a subject B will be described by using FIG. 13 and FIG. 15. FIG. 13 is a diagram illustrating another example of the gaze point with respect to the evaluation purpose image illustrated in FIG. 8. FIG. 15 is a diagram illustrating another example of the gaze point with respect to the evaluation purpose image illustrated in FIG. 10.

The evaluation value ANS1 of the subject B with respect to the evaluation purpose image illustrated in FIG. 8 is calculated to be ANS1=1+1+1+1+1+1+0=6.

The evaluation value ANS2 of the subject B with respect to the evaluation purpose image illustrated in FIG. 10 is calculated to be ANS2=1+1+1=3.

Based on these, the evaluation value ANS of the subject B is calculated to be ANS=6+3=9.

For example, if a threshold for determining whether or not a possibility of ASD is high is set to be "7", since the evaluation value ANS of the subject B is greater than or equal to the threshold, it is determined that "a possibility of ASD is low".

Furthermore, the evaluation value may also be calculated by applying weighting to the determination values of the determination areas.

A first method for performing weighting is a method for performing weighting in each of the determination areas included in the same image. For example, weighting is performed with respect to the determination areas that are set in the natural picture. In this case, the evaluation value ANS1 and the evaluation value ANS2 are represented as below. Furthermore, K denotes a coefficient of the weighting. The coefficient K can appropriately be set.

$$ANS1=X[A11]\cdot K+X[A12]\cdot K+X[A13]\cdot K+X[A14]+X[A15]+X[A16]+X[A17]$$

$$ANS2=X[A21]+X[A22]\cdot K+X[A23]\cdot K$$

Here, if the coefficient K is assumed to be "2", the evaluation value of the subject A is calculated as below.

$$ANS1=2+0+2+1+0+0+0=5$$

$$ANS2=1+0+2=3$$

ANS=8

Performing weighting, in other words, a conceivable method for selecting a determination area to be emphasized may include a selection method performed based on already known clinical findings, a selection method performed such that a diagnostic sensitivity becomes high based on the measurement result, or the like. Furthermore, it may also be possible to set a different value in each of the determination areas as the value of the coefficient K.

A second method for performing weighting is a method for performing weighting for the evaluation purpose images. For example, weighting is performed on the evaluation purpose image illustrated in FIG. 10. In this case, the evaluation value ANS1 and the evaluation value ANS2 are represented as below.

$$ANS1=X[A11]+X[A12]+X[A13]+X[A14]+X[A15]+X[A16]+X[A17]$$

$$ANS2=(X[A21]+X[A22]+X[A23])\cdot K$$

Here, if the coefficient K is assumed to be "2", the evaluation value of the subject A is calculated as below.

ANS1=3
ANS2=4
ANS=7

Performing weighting, in other words, a conceivable method for selecting an evaluation purpose image to be emphasized may include a selection method performed based on already known clinical findings, a selection method performed such that a diagnostic sensitivity becomes high based on the measurement result, or the like. Furthermore, it may also be possible to set a different value in each of the evaluation purpose images as the value of the coefficient K.

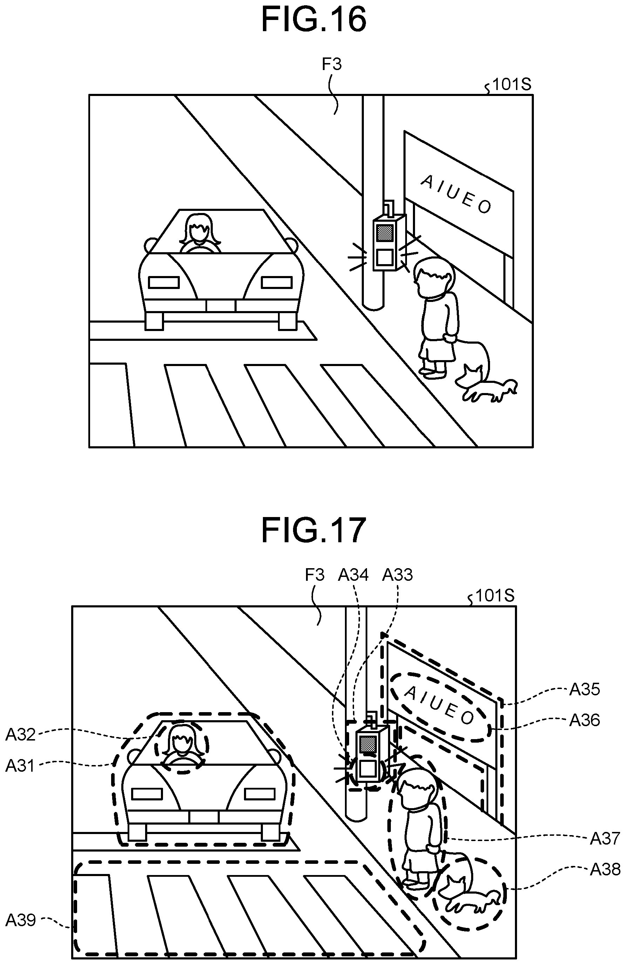

Another example of the evaluation purpose image will be described by using FIG. 16 and FIG. 17. FIG. 16 is a diagram illustrating another example of the evaluation purpose image. FIG. 17 is a diagram illustrating an example of the determination area that is set in the evaluation purpose image illustrated in FIG. 16. The evaluation purpose image is not limited to the image that includes the natural picture and the geometric image. The evaluation purpose image may also be a single natural picture that includes multiple objects.

The evaluation purpose image illustrated in FIG. 16 is a natural picture F3 that includes multiple objects. The natural picture F3 includes, for example, an automobile, a driver of the automobile, a traffic signal, a green light, a signboard, characters written on the signboard, a pedestrian, an animal, a pedestrian crossing, and the like.

Furthermore, as illustrated in FIG. 17, in order to perform the evaluation, in the evaluation purpose image, a determination area A31 is set to the automobile, a determination area A32 is set to the driver of the automobile, a determination area A33 is set to the traffic signal, a determination area A34 is set to the green light, a determination area A35 is set to the signboard, a determination area A36 is set to the characters written on the signboard, a determination area A37 is set to the pedestrian, a determination area A38 is set to the animal, and a determination area A39 is set to the pedestrian crossing. Furthermore, the determination area A32 is set inside the determination area A31, the determination area A34 is set inside the determination area A33, and the determination area A36 is set inside the determination area A35. In this case, when it is determined that the gaze point P is present inside the determination area A32, the determination area A34, and the determination area A36, it is determined that the gaze point P is also present inside the determination area A31, the determination area A33, and the determination area A35 respectively.

The evaluation unit 224 determines a possibility of ASD by determining whether the evaluation value ANS that is the evaluation data is greater than or equal to a predetermined threshold. For example, when the evaluation value ANS is greater than or equal to the threshold, it is possible to evaluate that the subject is less likely to be ASD. Furthermore, when the evaluation value ANS is not greater than or equal to the predetermined threshold, it is possible to evaluate that the subject is highly likely to be ASD.

In the embodiment, when the evaluation unit 224 outputs an evaluation result, the output controller 226 allows the output device 50 to output, in accordance with the evaluation result, character data indicating that, for example, "it seems that the subject is less likely to be a person with a developmental disability", character data indicating that "it seems that the subject is highly likely to be a person with a developmental disability", or the like.

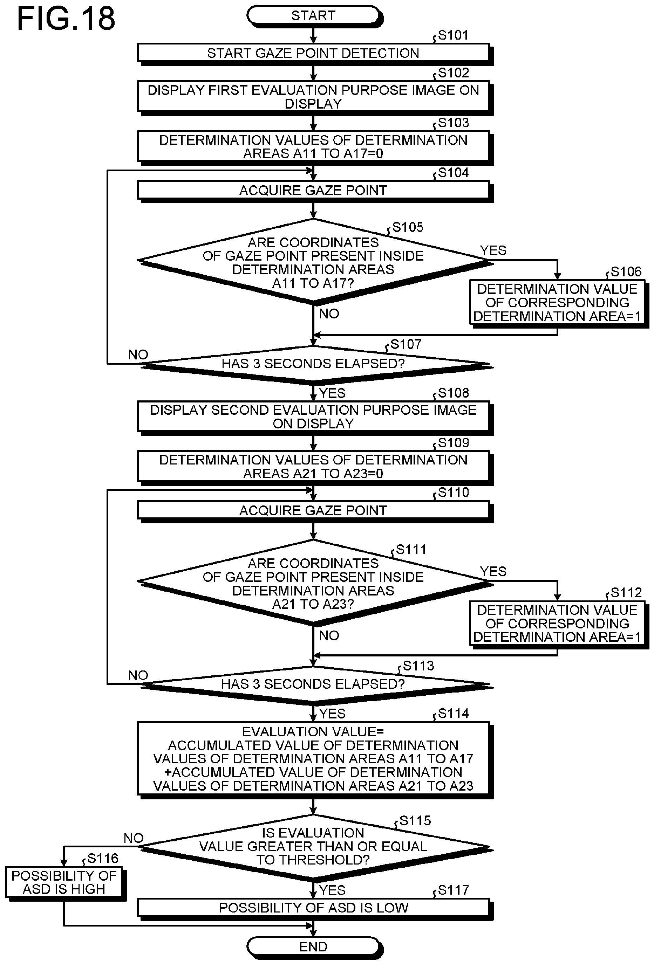

In the following, an example of the evaluation method according to the embodiment will be described with reference to FIG. 18. FIG. 18 is a flowchart illustrating an example of the evaluation method according to the embodiment.

The gaze point detecting unit 214 starts a gaze point detection (Step S101). Then, the process proceeds to Step S102.

The display controller 202 allows the display 101S to display a first evaluation purpose image (Step S102). In the embodiment, the display controller 202 allows the display 101S to display the evaluation purpose image illustrated in FIG. 8 Then, the process proceeds to Step S103.

The arithmetic unit 220 defines the determination values of the determination areas that have been set to the first evaluation purpose image to "0" (Step S103). In the embodiment, the arithmetic unit 220 defines the determination values of the determination area A11 to the determination area A17 that have been set in the first evaluation purpose image to "0". Then, the process proceeds to Step S104.

The gaze point detecting unit 214 acquires the gaze point P of the subject in the evaluation purpose image (Step S104). More specifically, the gaze point detecting unit 214 detects the positional data of the gaze point P of the subject on the display 101S included in the display device 101 for a predetermined sampling period (for example, 20 (msec)) while showing the first evaluation purpose image displayed on the display device 101 to the subject. Then, the process proceeds to Step S105.

The determination unit 218 determines whether coordinates of the gaze point P are present inside the determination area A11 to the determination area A17 (Step S105). More specifically, the determination unit 218 determines the determination area in which the gaze point P is present based on the positional data detected by the gaze point detecting unit 214 for the predetermined sampling period (for example, 20 (msec)).

When it is determined that the gaze point P is present inside the determination area A11 to the determination area A17 (Yes at Step S105), the determination unit 218 changes the determination value of the determination area that has been determined that the gaze point P is present to "1" (Step S106). Then, the process proceeds to Step S107.

When it is determined that the gaze point P is not present inside the determination area A11 to the determination area A17 (No at Step S105), the process proceeds to Step S107.

The arithmetic unit 220 determines, based on the detection result of the detection timer, whether or not the time reaches a completion time of a playback of the first evaluation purpose video (Step S107). In the embodiment, when 3 (sec) has elapsed from starting the playback of the first evaluation purpose video, the arithmetic unit 220 determines that the time reaches the completion time of the playback of the first evaluation purpose video. When it is determined, by the arithmetic unit 220, that the time reaches the completion time of the playback of the first evaluation purpose video (Yes at Step S107), the process proceeds to Step S108. In the embodiment, when 3 (sec) has not elapsed from starting of the playback of the first evaluation purpose video, the arithmetic unit 220 determines that the time does not reach the completion time of the playback of the first evaluation purpose video. When it is determined, by the arithmetic unit 220, that the time does not reach the completion time of the playback of the first evaluation purpose video (No at Step S107), the processes after Step S104 described above are repeatedly performed.

When it is determined, by the arithmetic unit 220, that the time reaches the completion time of the playback of the first evaluation purpose video (Yes at Step S107), the display controller 202 displays the second evaluation purpose image on the display 101S (Step S108). In the embodiment, the display controller 202 displays the evaluation purpose image illustrated in FIG. 10 on the display 101S. Then, the process proceeds to Step S109.

The arithmetic unit 220 defines the determination values of the determination areas that have been set to the second evaluation purpose image to "0" (Step S109). In the embodiment, the arithmetic unit 220 defines the determination values of the determination area A21 to the determination area A23 that have been set to the second evaluation purpose image to "0". Then, the process proceeds to Step S110.

The gaze point detecting unit 214 acquires the gaze point P of the subject in the evaluation purpose image (Step S110). More specifically, the gaze point detecting unit 214 detects the positional data of the gaze point P of the subject on the display 101S included in the display device 101 for a predetermined sampling period (for example, 20(msec)) while showing the second evaluation purpose image displayed on the display device 101. Then, the process proceeds to Step Sill.

The determination unit 218 determines whether the coordinate of the gaze point P are present inside the determination area A21 to the determination area A23 (Step Sill). More specifically, the determination unit 218 determines the determination area in which the gaze point P is present based on the positional data detected by the gaze point detecting unit 214 for the predetermined sampling period (for example 20(msec)).

When it is determined that the gaze point P is present inside the determination area A21 to the determination area A23 (Yes at Step S111), the determination unit 218 changes the determination value of the determination area that has been determined that the gaze point P is present to "1" (Step S112). Then, the process proceeds to Step S113.

When it is determined that the gaze point P is not present inside the determination area A21 to the determination area A23 (No at Step S111), the process proceeds to Step S113.

The arithmetic unit 220 determines, based on the detection result of the detection timer, whether the time reaches the completion time of a playback of the second evaluation purpose video (Step S113). In the embodiment, when 3 (sec) has elapsed from starting the playback of the second evaluation purpose video, the arithmetic unit 220 determines that it is time to complete the playback of the second evaluation purpose video. When it is determined, by the arithmetic unit 220, that the time reaches the completion time of the playback of the second evaluation purpose video (Yes at Step S113), the process proceeds to Step S114. In the embodiment, when 3 (sec) has not elapsed from starting the playback of the second evaluation purpose video, the arithmetic unit 220 determines that the time does not reach the completion time of the playback of the second evaluation purpose video. When it is determined, by the arithmetic unit 220, that the time does not reach the completion time of the playback of the second evaluation purpose video (No at Step S113), the processes after Step S110 described above are repeatedly performed.

The evaluation unit 224 calculates the evaluation value of the subject based on the total number of the determination areas in each of which the gaze point P is present (Step S114). The arithmetic unit 220 calculates the evaluation value ANS1 by accumulating the determination values for the first evaluation purpose image.

Furthermore, the arithmetic unit 220 calculates the evaluation value ANS2 by accumulating the determination values for the second evaluation purpose image. The evaluation unit 224 calculates the evaluation value ANS of the subject by adding the evaluation value ANS1 for the first evaluation purpose image and the evaluation value ANS2 for the second evaluation purpose image. Then, the process proceeds to Step S115.

The evaluation unit 224 determines whether the calculated evaluation value ANS is greater than or equal to the threshold (Step S115). In the embodiment, the evaluation unit 224 determines whether the evaluation value ANS is greater than or equal to the threshold of "7".

When the evaluation unit 224 determines that the calculated evaluation value ANS is not greater than or equal to the threshold (No at Step S115), the evaluation unit 224 evaluates that the subject is "highly likely to be ASD" (Step S116). After that, the output controller 226 outputs the evaluation result determined by the evaluation unit 224 and ends the processes.

When the evaluation unit 224 determines that the calculated evaluation value ANS is greater than or equal to the threshold (Yes at Step S115), the evaluation unit 224 evaluates that the subject is "less likely to be ASD" (Step S117). After that, the output controller 226 outputs the evaluation result determined by the evaluation unit 224 and ends the processes.

As described above, the evaluation device according to the embodiment includes: a display 101S configured to display at least one evaluation purpose image; a gaze point detecting unit 214 configured to detect positional data of a gaze point P of a subject who observes the display 101S; an area setting unit 216 configured to set at least one determination area in a position corresponding to the at least one evaluation purpose image on the display 101S; a determination unit 218 configured to determine, based on the positional data of the gaze point P, whether or not the gaze point P is present inside the at least one determination area to define at least one determination value for the at least one determination area; an arithmetic unit 220 configured to calculate, based on the at least one determination value defined by the determination unit 218, a number of the at least one determination area in which the gaze point P is present for a predetermined period; and an evaluation unit 224 configured to obtain evaluation data for evaluating a possibility that the subject is a person with a developmental disability based on the number, calculated by the arithmetic unit 220, of the at least one determination area in which the gaze point P is present.

Furthermore, the evaluation method according to the embodiment includes: a displaying step of displaying at least one evaluation purpose image on a display 101S; a gaze point detecting step of detecting positional data of a gaze point P of a subject who observes the display 101S; an area setting step of setting at least one determination area in a position corresponding to the at least one evaluation purpose image on the display 101S; a determination step of determining, based on the positional data of the gaze point P, whether or not the gaze point P is present inside the at least one determination area to define at least one determination value for the at least one determination area; an arithmetic step of calculating, based on the at least one determination value defined at the determination step, a number of the at least one determination area in which the gaze point P is present for a predetermined period; and an evaluation step of obtaining evaluation data for evaluating a possibility that the subject is a person with a developmental disability based on the number, calculated at the arithmetic step, of the at least one determination area in which the gaze point p is present.

Furthermore, the evaluation program according to the embodiment causes a computer to execute: a displaying step of displaying at least one evaluation purpose image on a display 101S; a gaze point detecting step of detecting positional data of a gaze point P of a subject who observes the display 101S; an area setting step of setting at least one determination area in a position corresponding to the at least one evaluation purpose image on the display 101S; a determination step of determining, based on the positional data of the gaze point P, whether or not the gaze point P is present inside the at least one determination area to define at least one determination value for the at least one determination area; an arithmetic step of calculating, based on the at least one determination value defined at the determination step, a number of the at least one determination area in which the gaze point P is present for a predetermined period; and an evaluation step of obtaining evaluation data for evaluating a possibility that the subject is a person with a developmental disability based on the number, calculated at the arithmetic step, of the at least one determination area in which the gaze point p is present.

According to the embodiment, it is possible to obtain the evaluation data of the subject based on the total number of the determination areas in each of which the gaze point P is present, in other words, depending on how many determination areas in the evaluation purpose image has been looked at by the subject. According to the embodiment, it is possible to simply perform diagnosis support of ASD of the subject with high accuracy.

In the embodiment, the area setting unit 216 sets multiple determination areas to a person, an object, a pattern, and the like included in the image. Since the subject with ASD tends to be interested in a specific object, the subjects is highly likely to gaze one of the multiple determination areas. In this way, according to the embodiment, it is possible to efficiently obtain highly accurate evaluation data.

In the embodiment, the evaluation unit 224 obtains the evaluation data by weighting for the determination area in which the gaze point P is present. Accordingly, it is possible to appropriately give a priority order to the evaluation values by using a selection method based on already known clinical findings, a selection method performed such that diagnostic sensitivity becomes high based on the measurement result, or the like. According to the embodiment, it is possible to obtain further highly accurate evaluation data.

In the embodiment, the evaluation unit 224 evaluates the possibility of ASD the by determining whether the evaluation data is greater than or equal to the predetermined threshold. According to the embodiment, it is possible to easily obtain further highly accurate.

The technical scope of the present disclosure is not limited to the embodiments described above and various modifications are possible as long as they do not depart from the spirit of the present invention. For example, in each of the embodiments described above, a description has been given of a case, as an example, in which the line-of-sight detecting device 100 is used as the evaluation device that evaluates a possibility of ASD. However, the embodiment is not limited thereto. For example, the line-of-sight detecting device 100 may also be applicable to the evaluation device that evaluates a possibility of attention-deficit hyperactivity disorder (ADHD).

**Industrial Applicability**

It is possible to use the evaluation device, the evaluation method, and the evaluation program according to the present disclosure in, for example, the line-of-sight detecting device.

**Claims**

1.    An evaluation device (100) comprising:

a display (1015) configured to display at least one evaluation purpose image (F1, F2, F3, G1, G2);
a gaze point detecting unit (214) configured to detect positional data of a gaze point (P) of a subject who observes the display (1015);
an area setting unit (216) configured to set determination areas (A11 to A17, A21 to A23, A31 to A39) in positions corresponding to the at least one evaluation purpose image (F1, F2, F3, G1, G2) on the display (1015);
a determination unit (218) configured to determine, based on the positional data of the gaze point (P), whether or not the gaze point (P) is present inside each of the determination areas (A11 to A17, A21 to A23, A31 to A39) to define determination values (X[A11] to X[A17], X[A21] to X[A23], X[A31] to X[A39]) for determination areas (A11 to A17, A21 to A23, A31 to A39) respectively;
an arithmetic unit (220) configured to calculate, based on the determination values (X[A11] to X[A17], X[A21] to X[A23], X[A31] to X[A39]) defined by the determination unit (218), a number of the determination areas (A11 to A17, A21 to A23, A31 to A39) in which the gaze point (P) is present for a predetermined period; and
an evaluation unit (224) configured to obtain evaluation data (ANS, ANS1, ANS2) for evaluating a possibility that the subject is a person with a developmental disability based on the number, calculated by the arithmetic unit (220), of the determination areas (A11 to A17, A21 to A23, A31 to A39) in which the gaze point (P) is present, wherein
the evaluation unit (224) is further configured to obtain the value of the evaluation data (ANS, ANS1, ANS2) of the subject based on an equation which calculates a larger value of the evaluation data (ANS, ANS1, ANS2) as the number of the determination areas (A11 to A17, A21 to A23, A31 to A39) in which the gaze point (P) is present becomes larger, and to evaluate that the possibility that the subject is the person with the developmental disability is lower as the value of the evaluation data (ANS, ANS1, ANS2) becomes larger.

2.    The evaluation device (100) according to claim 1, wherein the evaluation unit (224) is further configured to perform the evaluation by determining whether the value of the evaluation data (ANS, ANS1, ANS2) is greater than or equal to a predetermined threshold.

3. The evaluation device (100) according to claim 1 or 2, wherein
the area setting unit (216) is further configured to set the determination areas (A11 to A17, A21 to A23, A31 to A39) in regions in which a difference in a tendency to be gazed appears between the subject with a high possibility of ASD (Autism Spectrum Disorder) and the subject with a low possibility of ASD.

4. The evaluation device (100) according to any one of claims 1 to 3, wherein

the evaluation purpose image includes a natural image (F1, F2) and a geometric image (G1, G2);
the area setting unit (216) is further configured to set the determination areas (A11 to A13, A22 to A23) in at least one part of a region in which the natural image (F1, F2) is displayed and set the determination areas (A14 to A17, A21) in at least one part of a region in which the geometric image (G1, G2) is displayed respectively; and
the evaluation unit (224) is further configured to calculate the value of the evaluation data (ANS, ANS1, ANS2) by weighting the determination values (X[A11] to X[A17], X[A21] to X[A23]) such that the determination values (X[A11] to X[A13], X[A22] to X[A23]) for the determination areas (A11 to A13, A22 to A23) set in the natural image (F1, F2) becomes larger than the determination values (X[A14] to X[A17], X[A21]) for the determination areas (A14 to A17, A21) set in the geometric image (G1, G2) and accumulating the determination values for the determination areas (X[A11] to X[A17], X[A21] to X[A23]) defined in the natural image (F1, F2) and the geometric image (G1, G2).

5. An evaluation method comprising:

a displaying step of displaying at least one evaluation purpose image (F1, F2, F3, G1, G2) on a display (1015);
a gaze point detecting step of detecting positional data of a gaze point (P) of a subject who observes the display (101S);
an area setting step of setting determination areas (A11 to A17, A21 to A23, A31 to A39) in positions corresponding to the at least one evaluation purpose image (F1, F2, F3, G1, G2) on the display (1015);
a determination step of determining, based on the positional data of the gaze point (P), whether or not the gaze point (P) is present inside each of the determination areas (A11 to A17, A21 to A23, A31 to A39) to define determination values (X[A11] to X[A17], X[A21] to X[A23], X[A31] to X[A39]) for the determination areas (A11 to A17, A21 to A23, A31 to A39) respectively;
an arithmetic step of calculating, based on the at least one determination value (X[A11] to X[A17], X[A21] to X[A23], X[A31] to X[A39]) defined at the determination step, a number of the determination areas (A11 to A17, A21 to A23, A31 to A39) in which the gaze point (P) is present for a predetermined period; and
an evaluation step of obtaining evaluation data (ANS, ANS1, ANS3) for evaluating a possibility that the subject is a person with a developmental disability based on the number, calculated at the arithmetic step, of the determination areas (A11 to A17, A21 to A23, A31 to A39) in which the gaze point (P) is present, wherein
the evaluation step further comprises obtaining the value of the evaluation data (ANS, ANS1, ANS2) of the subject based on an equation which calculates a larger value of the evaluation data (ANS, ANS1, ANS2) as the number of the determination areas (A11 to A17, A21 to A23, A31 to A39) in which the gaze point (P) is present becomes larger, and to evaluate that the possibility that the subject is the person with the developmental disability is lower as the value of the evaluation data (ANS, ANS1, ANS2) becomes larger.

6. An evaluation program that causes a computer to execute:

a displaying step of displaying at least one evaluation purpose image (F1, F2, F3, G1, G2) on a display (1015);
a gaze point detecting step of detecting positional data of a gaze point (P) of a subject who observes the display (101S);
an area setting step of setting determination areas (A11 to A17, A21 to A23, A31 to A39) in positions corresponding to the at least one evaluation purpose image (F1, F2, F3, G1, G2) on the display (1015);
a determination step of determining, based on the positional data of the gaze point (P), whether or not the gaze point (P) is present inside each of the determination areas (A11 to A17, A21 to A23, A31 to A39) to define determination values (X[A11] to X[A17], X[A21] to X[A23], X[A31] to X[A39]) for the determination areas (A11 to A17, A21 to A23, A31 to A39) respectively;
an arithmetic step of calculating, based on the determination values (X[A11] to X[A17], X[A21] to X[A23], X[A31] to X[A39]) defined at the determination step, a number of the determination areas (A11 to A17, A21 to A23, A31 to A39) in which the gaze point (P) is present for a predetermined period; and
an evaluation step of obtaining evaluation data (ANS, ANS1, ANS3) for evaluating a possibility that the subject is a person with a developmental disability based on the number, calculated at the arithmetic step, of the determination areas (A11 to A17, A21 to A23, A31 to A39) in which the gaze point (P) is present, wherein the evaluation step further comprises obtaining the value of the evaluation data (ANS, ANS1, ANS2) of the subject based on an equation which calculates a larger value of the evaluation data (ANS, ANS1, ANS2) as the number of the determination areas (A11 to A17, A21 to A23, A31 to A39) in which the gaze point (P) is present becomes larger, and to evaluate that the possibility that the subject is the person with the developmental disability is lower as the value of the evaluation data (ANS, ANS1, ANS2) becomes larger.

**Patentansprüche**

1. Beurteilungsvorrichtung (100), die Folgendes aufweist:

   eine Anzeige (101S), die konfiguriert ist, um zumindest ein Beurteilungszweckbild (F1, F2, F3, G1, G2) anzuzeigen;
   eine Blickpunktdetektionseinheit (214), die konfiguriert ist, um Positionsdaten eines Blickpunktes (P) einer Person zu detektieren, welche die Anzeige (101S) betrachtet;
   eine Bereichsfestlegungseinheit (216), die konfiguriert ist, um Bestimmungsbereiche (A11 bis A17, A21 bis A23, A31 bis A39) an Positionen entsprechend dem mindestens einen Beurteilungszweckbild (F1, F2, F3, G1, G2) auf der Anzeige (101S) festzulegen;
   wobei die Bestimmungseinheit (218) konfiguriert ist, um basierend auf den Positionsdaten des Blickpunktes (Patentdokument) zu bestimmen, ob der Blickpunkt (P) in jedem der Bestimmungsbereiche (A11 bis A17, A21 bis A23, A31 bis A39) vorhanden ist oder nicht, um Bestimmungswerte (X[A11] bis X[A17], X[A21] bis X[A23], X[A31] bis X[A39] für entsprechende Bestimmungsbereiche (A11 bis A17, A21 bis A23, A31 bis A39) zu definieren;
   eine Arithmetikeinheit (220), die konfiguriert ist, um basierend auf den Bestimmungswerten (X[A11] bis X[A17], X[A21] bis X[A23], X[A31] bis X[A39], die durch die Bestimmungseinheit (218) definiert wurden, eine Anzahl von Bestimmungsbereichen (A11 bis A17, A21 bis A23, A31 bis A39) zu berechnen, in welchen der Blickpunkt (P) für eine vorbestimmte Periode vorhanden ist; und
   eine Beurteilungseinheit (224, die konfiguriert ist, um Beurteilungsdaten (ANS, ANS1, ANS2) zu erlangen, um eine Möglichkeit zu beurteilen, dass die Person eine Person mit einer Entwicklungsunfähigkeit ist, und zwar basierend auf der von der Arithmetikeinheit (220) berechneten Anzahl der Bestimmungsbereiche (A11 bis A17, A21 bis A23, A31 bis A39), in denen der Blickpunkt (P) vorhanden ist, wobei
   die Beurteilungseinheit (224) weiter konfiguriert ist, um den Wert der Beurteilungsdaten (ANS, ANS1, ANS2) der Person basierend auf einer Gleichung zu erlangen, welche einen größeren Wert der Beurteilungsdaten (ANS, ANS1, ANS2) berechnet, wenn die Anzahl der Bestimmungsbereiche (A11 bis A17, A21 bis A23, A31 bis A39), in denen der Blickpunkt (P) vorhanden ist, größer wird, und um zu beurteilen, dass die Möglichkeit, dass die Person eine Person mit der Entwicklungsunfähigkeit ist, geringer ist, wenn der Wert der Beurteilungsdaten (ANS, ANS1, ANS2) größer wird.

2. Beurteilungsvorrichtung (100) nach Anspruch 1, wobei die Beurteilungseinheit (224) weiter konfiguriert ist, um die Beurteilung auszuführen, indem sie bestimmt, ob der Wert der Beurteilungsdaten (ANS, ANS1, ANS2) größer oder gleich einer vorbestimmten Schwelle ist.

3. Beurteilungsvorrichtung (100) nach Anspruch 1 oder 2, wobei
   die Bereichsfestlegungseinheit (216) weiter konfiguriert ist, um die Bestimmungsbereiche (A11 bis A17, A21 bis A23, A31 bis A39) in Regionen festzulegen, in welchen ein Unterschied bei einer Tendenz angeschaut zu werden, zwischen der Person mit einer hohen Möglichkeit von ASD, Autismus Spectrum Disorder, Autismus Spektrum Störung, und der Person mit einer niedrigen Wahrscheinlichkeit von ASD erscheint.

4. Beurteilungsvorrichtung (100) nach einem der Ansprüche 1 bis 3, wobei

   das Beurteilungszweckbild ein natürliches Bild (F1, F2) und ein geometrisches Bild (G1, G2) aufweist;
   die Bereichsfestlegungseinheit (216) weiter konfiguriert ist, um die Bestimmungsbereiche (A11 bis A17, A21 bis A23) in mindestens einem Teil einer Region festzulegen, in welcher das natürliche Bild (F1, F2) angezeigt wird, und die Bestimmungsbereiche (A14 bis A17, A21) in mindestens einem Teil einer Region festzulegen, in welchem entsprechend das geometrische Bild (G1, G2) angezeigt wird; und
   wobei die Beurteilungseinheit (224) weiter konfiguriert ist, um den Wert der Beurteilungsdaten (ANS, ANS1, ANS2) durch Gewichtung der Bestimmungswerte (X[A11] bis X[A17], X[A21] bis X[A23]) so zu berechnen, dass die Bestimmungswerte (X[A11] bis X[A13], X[A22] bis X[A23]) für die Bestimmungsbereiche (A11 bis A13, A22 bis A23), die in dem natürlichen Bild (F1, F2) festgelegt sind, größer werden als die Bestimmungswerte (X[A14] bis X[A17], X[A21]) für die Bestimmungsbereiche (A14 bis A17, A21), die in dem geometrischen Bild (G1, G2) festgelegt sind, und die Bestimmungswerte für die Bestimmungsbereiche (X[A11] bis X[A17], X[A21] bis X[A23]) zu akkumulieren, die in dem natürlichen Bild (F1, F2) und dem geometrischen Bild (G1, G2) definiert sind.

5. Beurteilungsverfahren, welches Folgendes aufweist:

einen Anzeigeschritt zum Anzeigen von mindestens einem Beurteilungszweckbild (F1, F2, F3, G1, G2) auf einer Anzeige (101S);
einen Blickpunktdetektionsschritt des Detektierens von Positionsdaten eines Blickpunktes (P) einer Person, welche die Anzeige (101S) betrachtet;
einen Bereichsfestlegungsschritt des Festlegens von Bestimmungsbereichen (A11 bis A17, A21 bis A23, A31 bis A39) an Positionen entsprechend dem mindestens einen Beurteilungszweckbild (F1, F2, F3, G1, G2) auf der Anzeige (101S);
einen Bestimmungsschritt des Bestimmens, und zwar basierend auf den Positionsdaten des Blickpunktes (P), ob der Blickpunkt (P) in jedem der Bestimmungsbereiche (A11 bis A17, A21 bis A23, A31 bis A39) vorhanden ist, oder nicht, um Bestimmungswerte (X[A11] bis X[A17], X[A21] bis X[A23]), (X[A31] bis X[A39]) für die entsprechenden Bestimmungsbereiche (A11 bis A17, A21 bis A23, A31 bis A39) zu definieren;
einen Arithmetikschritt zum Berechnen, und zwar basierend auf dem mindestens einen Bestimmungswert (X[A11] bis X[A17], X[A21] bis X[A23]), (X[A31] bis X[A39]), der in dem Bestimmungsschritt definiert wurde, einer Anzahl der Bestimmungsbereiche (A11 bis A17, A21 bis A23, A31 bis A39), in welchen der Blickpunkt (P) für eine vorbestimmte Periode vorhanden ist; und
einen Beurteilungsschritt des Erlangens von Beurteilungsdaten (ANS, ANS1, ANS3) zum Beurteilen einer Möglichkeit, dass die Person eine Person mit einer Entwicklungsunfähigkeit ist, und zwar basierend auf der in dem Arithmetikschritt berechneten Anzahl der Bestimmungsbereiche (A11 bis A17, A21 bis A23, A31 bis A39), in welchen der Blickpunkt (P) vorhanden ist, wobei
der Beurteilungsschritt weiter aufweist, den Wert der Beurteilungsdaten (ANS, ANS1, ANS2) der Person, basierend auf einer Gleichung zu erlangen, welche einen größeren Wert der Beurteilungsdaten (ANS, ANS1, ANS2) berechnet, wenn die Anzahl der Bestimmungsbereiche (A11 bis A17, A21 bis A23, A31 bis A39), in welchen der Blickpunkt (P) vorhanden ist, größer wird, und zu beurteilen, dass die Wahrscheinlichkeit, dass die Person die Person mit der Entwicklungsunfähigkeit ist, geringer ist, wenn der Wert der Beurteilungsdaten (ANS, ANS1, ANS2) größer wird.

6. Beurteilungsprogramm, welches einen Computer veranlasst, Folgendes auszuführen:
einen Anzeigeschritt zum Anzeigen von mindestens einem Beurteilungszweckbild (F1, F2, F3, G1, G2) auf einer Anzeige (101S);

einen Blickpunktdetektionsschritt des Detektierens von Positionsdaten eines Blickpunktes (P) einer Person, welche die Anzeige (101S) betrachtet;
einen Bereichsfestlegungsschritt des Festlegens von Bestimmungsbereichen (A11 bis A17, A21 bis A23, A31 bis A39) an Positionen entsprechend dem mindestens einen Beurteilungszweckbild (F1, F2, F3, G1, G2) auf der Anzeige (101S);
einen Bestimmungsschritt des Bestimmens, und zwar basierend auf den Positionsdaten des Blickpunktes (P), ob der Blickpunkt (P) in jedem der Bestimmungsbereiche (A11 bis A17, A21 bis A23, A31 bis A39) vorhanden ist, oder nicht, um Bestimmungswerte (X[A11] bis X[A17], X[A21] bis X[A23]), (X[A31] bis X[A39]) für die entsprechenden Bestimmungsbereiche (A11 bis A17, A21 bis A23, A31 bis A39) zu definieren;
einen Arithmetikschritt zum Berechnen, und zwar basierend auf dem mindestens einen Bestimmungswert (X[A11] bis X[A17], X[A21] bis X[A23]), (X[A31] bis X[A39]), der in dem Bestimmungsschritt definiert wurde, einer Anzahl der Bestimmungsbereiche (A11 bis A17, A21 bis A23, A31 bis A39), in welchen der Blickpunkt (P) für eine vorbestimmte Periode vorhanden ist; und
einen Beurteilungsschritt des Erlangens von Beurteilungsdaten (ANS, ANS1, ANS3) zum Beurteilen einer Möglichkeit, dass die Person eine Person mit einer Entwicklungsunfähigkeit ist, und zwar basierend auf der in dem Arithmetikschritt berechneten Anzahl der Bestimmungsbereiche (A11 bis A17, A21 bis A23, A31 bis A39), in welchen der Blickpunkt (P) vorhanden ist, wobei
der Beurteilungsschritt weiter aufweist, den Wert der Beurteilungsdaten (ANS, ANS1, ANS2) der Person, basierend auf einer Gleichung zu erlangen, welche einen größeren Wert der Beurteilungsdaten (ANS, ANS1, ANS2) berechnet, wenn die Anzahl der Bestimmungsbereiche (A11 bis A17, A21 bis A23, A31 bis A39), in welchen der Blickpunkt (P) vorhanden ist, größer wird, und zu beurteilen, dass die Wahrscheinlichkeit, dass die Person die Person mit der Entwicklungsunfähigkeit ist, geringer ist, wenn der Wert der Beurteilungsdaten (ANS, ANS1, ANS2) größer wird.

**Revendications**

1. Dispositif d'évaluation (100) comprenant :

   un écran (101S) configuré pour afficher au moins une image destinée à une évaluation (F1, F2, F3, G1, G2) ;
   une unité de détection de point de regard (214) configurée pour détecter des données de position d'un point de regard (P) d'un sujet qui observe l'affichage (101S) ;
   une unité de définition de zone (216) configurée pour définir des zones de détermination (A11 à A17, A21 à A23, A31 à A39) dans des emplacements correspondant à l'au moins une image destinée à une évaluation (F1, F2, F3, G1, G2) sur l'affichage (101S) ;
   une unité de détermination (218) configurée pour déterminer, sur la base des données de position du point de regard (P), si le point de regard (P) est présent ou non à l'intérieur de chacune des zones de détermination (A11 à A17, A21 à A23, A31 à A39) pour définir des valeurs de détermination (X[A11] à X[A17], X[A21] à X[A23], X[A31] à X[A39]) pour des zones de détermination (A11 à A17, A21 à A23, A31 à A39) respectivement ;
   une unité arithmétique (220) configurée pour calculer, sur la base des valeurs de détermination (X[A11] à X[A17], X[A21] à X[A23], X[A31] à X[A39]) définies par l'unité de détermination (218), un nombre total des zones de détermination (A11 à A17, A21 à A23, A31 à A39) dans lesquelles le point de regard (P) est présent pendant une période prédéterminée ; et
   une unité d'évaluation (224) configurée pour obtenir des données d'évaluation (ANS, ANS1, ANS2) pour évaluer une possibilité que le sujet soit une personne ayant une déficience intellectuelle sur la base du nombre, calculé par l'unité arithmétique (220), des zones de détermination (A11 à A17, A21 à A23, A31 à A39) dans lesquelles le point de regard (P) est présent, dans lequel
   l'unité d'évaluation (224) est configurée en outre pour obtenir la valeur des données d'évaluation (ANS, ANS1, ANS2) du sujet sur la base d'une équation qui calcule une valeur des données d'évaluation (ANS, ANS1, ANS2) plus grande lorsque le nombre des zones de détermination (A11 à A17, A21 à A23, A31 à A39) dans lesquelles le point de regard (P) est présent devient plus grand, et pour évaluer que la possibilité que le sujet soit une personne ayant une déficience intellectuelle est plus petite lorsque la valeur des données d'évaluation (ANS, ANS1, ANS2) devient plus grande.

2. Dispositif d'évaluation (100) selon la revendication 1, dans lequel l'unité d'évaluation (224) est en outre configurée pour effectuer l'évaluation en déterminant si la valeur des données d'évaluation (ANS, ANS1, ANS2) est supérieure ou égale à un seuil prédéterminé.

3. Dispositif d'évaluation (100) selon la revendication 1 ou 2, dans lequel
   l'unité d'évaluation (224) est en outre configurée pour définir les zones de détermination (A11 à A17, A21 à A23, A31 à A39) dans des régions dans lesquelles une différence dans une tendance à être regardé apparait entre le sujet ayant une possibilité élevée d'avoir un ASD (trouble du spectre autistique) et le sujet ayant une faible possibilité d'ASD.

4. Dispositif d'évaluation (100) selon l'une quelconque des revendications 1 à 3, dans lequel

   l'image destinée à une évaluation comporte une image naturelle (F1, F2) et une image géométrique (G1, G2) ;
   l'unité de définition de zone (216) est configurée en outre pour définir les zones de détermination (A11 à A13, A22 à A23) dans au moins une partie d'une région dans laquelle l'image naturelle (F1, F2) est affichée et pour définir les zones de détermination (A14 à A17, A21) dans au moins une partie d'une région dans laquelle l'image géométrique (G1, G2) est affichée, respectivement ; et
   l'unité d'évaluation (224) est configurée en outre pour calculer la valeur des données d'évaluation (ANS, ANS1, ANS2) en pondérant les valeurs de détermination (X[A11] à X[A17], X[A21] à X[A23]) de sorte que les valeurs de détermination (X[A11] à X[A17], X[A21] à X[A23]) pour les zones de détermination (A11 à A13, A22 à A23) définies dans l'image naturelle (F1, F2) deviennent plus grande que les valeurs de détermination (X[A14] à X[A17], X[A21]) pour les zones de détermination (A14 à A17, A21) définies dans l'image géométrique (G1, G2) et en cumulant les valeurs de détermination pour les valeurs de détermination (X[A11] à X[A17], X[A21] à X[A23]) définies dans l'image naturelle (F1, F2) et dans l'image géométrique (G1, G2).

**5.** Procédé d'évaluation comprenant :

une étape d'affichage d'affichage d'au moins une image destinée à une évaluation (F1, F2, F3, G1, G2) sur un affichage (101S) ;
une étape de détection de point de regard de détection de données de position d'un point de regard (P) d'un sujet qui observe l'affichage (101S) ;
une étape de définition de zone de définition de zones de détermination (A11 à A17, A21 à A23, A31 à A39) dans des emplacements correspondant à l'au moins une image destinée à une évaluation (F1, F2, F3, G1, G2) sur l'affichage (101S) ;
une étape de détermination de détermination, sur la base des données de position du point de regard (P), si le point de regard (P) est présent ou non à l'intérieur de chacune des zones de détermination (A11 à A17, A21 à A23, A31 à A39) pour définir des valeurs de détermination (X[A11] à X[A17], X[A21] à X[A23], X[A31] à X[A39]) pour des zones de détermination (A11 à A17, A21 à A23, A31 à A39) respectivement ;
une étape arithmétique de calcul, sur la base des au moins une valeur de détermination (X[A11] à X[A17], X[A21] à X[A23], X[A31] à X[A39]) définies à l'étape de détermination, d'un nombre des zones de détermination (A11 à A17, A21 à A23, A31 à A39) dans lesquelles le point de regard (P) est présent pendant une période prédéterminée ; et
une étape d'évaluation d'obtention de données d'évaluation (ANS, ANS1, ANS2) pour évaluer une possibilité que le sujet soit une personne ayant une déficience intellectuelle sur la base du nombre, calculé à l'étape arithmétique (220), des zones de détermination (A11 à A17, A21 à A23, A31 à A39) dans lesquelles le point de regard (P) est présent, dans lequel
l'étape d'évaluation comprend en outre l'obtention de la valeur des données d'évaluation (ANS, ANS1, ANS2) du sujet sur la base d'une équation qui calcule une valeur des données d'évaluation (ANS, ANS1, ANS2) plus grande lorsque le nombre des zones de détermination (A11 à A17, A21 à A23, A31 à A39) dans lesquelles le point de regard (P) est présent devient plus grand, et pour évaluer que la possibilité que le sujet soit une personne ayant une déficience intellectuelle est plus petite lorsque la valeur des données d'évaluation (ANS, ANS1, ANS2) devient plus grande.

**6.** Programme d'évaluation qui amène un ordinateur à exécuter :

une étape d'affichage d'affichage d'au moins une image destinée à une évaluation (F1, F2, F3, G1, G2) sur un affichage (101S) ;
une étape de détection de point de regard de détection de données de position d'un point de regard (P) d'un sujet qui observe l'affichage (101S) ;
une étape de définition de zone de définition de zones de détermination (A11 à A17, A21 à A23, A31 à A39) dans des emplacements correspondant à l'au moins une image destinée à une évaluation (F1, F2, F3, G1, G2) sur l'affichage (101S) ;
une étape de détermination de détermination, sur la base des données de position du point de regard (P), si le point de regard (P) est présent ou non à l'intérieur de chacune des zones de détermination (A11 à A17, A21 à A23, A31 à A39) pour définir des valeurs de détermination (X[A11] à X[A17], X[A21] à X[A23], X[A31] à X[A39]) pour les zones de détermination (A11 à A17, A21 à A23, A31 à A39) respectivement ;
une étape arithmétique de calcul, sur la base des valeurs de détermination (X[A11] à X[A17], X[A21] à X[A23], X[A31] à X[A39]) définies à l'étape de détermination, d'un nombre des zones de détermination (A11 à A17, A21 à A23, A31 à A39) dans lesquelles le point de regard (P) est présent pendant une période prédéterminée ; et
une étape d'évaluation d'obtention de données d'évaluation (ANS, ANS1, ANS2) pour évaluer une possibilité que le sujet soit une personne ayant une déficience intellectuelle sur la base du nombre, calculé à l'étape arithmétique (220), des zones de détermination (A11 à A17, A21 à A23, A31 à A39) dans lesquelles le point de regard (P) est présent, dans lequel
l'étape d'évaluation comprend en outre l'obtention de la valeur des données d'évaluation (ANS, ANS1, ANS2) du sujet sur la base d'une équation qui calcule une valeur des données d'évaluation (ANS, ANS1, ANS2) plus grande lorsque le nombre des zones de détermination (A11 à A17, A21 à A23, A31 à A39) dans lesquelles le point de regard (P) est présent devient plus grand, et pour évaluer que la possibilité que le sujet soit une personne ayant une déficience intellectuelle est plus petite lorsque la valeur des données d'évaluation (ANS, ANS1, ANS2) devient plus grande.

# FIG.1

# FIG.2

# FIG.3

100

| 20 | 30 | 40 |

**20**

**202**
DISPLAY CONTROLLER

**204**
LIGHT SOURCE CONTROLLER

**206**
IMAGE DATA ACQUIRING UNIT

**208**
INPUT DATA ACQUIRING UNIT

**210**
POSITION DETECTING UNIT

**212**
CURVERTURE CENTER CALCULATING UNIT

**214**
GAZE POINT DETECTING UNIT

**216**
AREA SETTING UNIT

**218**
DETERMINATION UNIT

**220**
ARITHMETIC UNIT

**222**
STORAGE

**224**
EVALUATION UNIT

**226**
OUTPUT CONTROLLER

**30**

**302**
INPUT/ OUTPUT UNIT

**40**

**402**
DISPLAY DEVICE DRIVING UNIT

**404A**
FIRST CAMERA INPUT/ OUTPUT UNIT

**404B**
SECOND CAMERA INPUT/ OUTPUT UNIT

**406**
LIGHT SOURCE DRIVING UNIT

**101**
DISPLAY DEVICE

**102**

**102A**
FIRST CAMERA

**102B**
SECOND CAMERA

**103**

**103A**
FIRST LIGHT SOURCE

**103B**
SECOND LIGHT SOURCE

**50**
OUTPUT DEVICE

**60**
INPUT DEVICE

# FIG.4

# FIG.5

# FIG.6

# FIG.7

# FIG.8

F1    G1    101S

# FIG.9

F1  A11    A14  G1    101S

A12
A13

A15

A16

A17

# FIG.10

G2   F2   101S

# FIG.11

G2   F2   101S

A21   A22

A23

# FIG.12

F1　A11　　　　　A14　G1　　　　101S

A12

A13

A15

A16

A17

# FIG.13

F1　A11　　　　　A14　G1　　　　101S

A12

A13

A15

A16

A17

# FIG.14

# FIG.15

# FIG.16

# FIG.17

# FIG.18

START

↓ S101

START GAZE POINT DETECTION

↓ S102

DISPLAY FIRST EVALUATION PURPOSE IMAGE ON DISPLAY

↓ S103

DETERMINATION VALUES OF DETERMINATION AREAS A11 TO A17=0

↓ S104

ACQUIRE GAZE POINT

↓ S105

ARE COORDINATES OF GAZE POINT PRESENT INSIDE DETERMINATION AREAS A11 TO A17? — YES → S106

DETERMINATION VALUE OF CORRESPONDING DETERMINATION AREA=1

NO ↓

S107

HAS 3 SECONDS ELAPSED? — NO

YES ↓ S108

DISPLAY SECOND EVALUATION PURPOSE IMAGE ON DISPLAY

↓ S109

DETERMINATION VALUES OF DETERMINATION AREAS A21 TO A23=0

↓ S110

ACQUIRE GAZE POINT

↓ S111

ARE COORDINATES OF GAZE POINT PRESENT INSIDE DETERMINATION AREAS A21 TO A23? — YES → S112

DETERMINATION VALUE OF CORRESPONDING DETERMINATION AREA=1

NO ↓

S113

HAS 3 SECONDS ELAPSED? — NO

YES ↓ S114

EVALUATION VALUE= ACCUMULATED VALUE OF DETERMINATION VALUES OF DETERMINATION AREAS A11 TO A17 +ACCUMULATED VALUE OF DETERMINATION VALUES OF DETERMINATION AREAS A21 TO A23

↓ S115

IS EVALUATION VALUE GREATER THAN OR EQUAL TO THRESHOLD? — NO → S116

POSSIBILITY OF ASD IS HIGH

YES ↓ S117

POSSIBILITY OF ASD IS LOW

↓

END

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2011206542 A
- WO 2018216347 A
- JP 2018140007 A
- JP 2014068932 A
- US 2016106354 A